# EUROPEAN PATENT APPLICATION

(11) **EP 4 624 483 A1**
(43) Date of publication of application: **01.10.2025**
(21) Application number: 23894024.1
(22) Date of filing: 24.11.2023
(51) Int. Cl.: C07F 15/00, A61K 31/555, A61P 35/00, A61K 33/243

(54) **GRAMINE-PLATINUM (IV) COMPLEX AS WELL AS PREPARATION METHOD THEREFOR AND ANTI-TUMOR USE THEREOF**

(30) Priority: 24.11.2022 CN 202211480367
(71) Applicant: Tianjin Medical University Cancer Institute and Hospital, Tianjin 300060 (CN); Tianjin Medical University, Tianjin 300070 (CN)
(72) Inventor: HAO, Jihui, Tianjin 300060 (CN); XU, Jingyuan, Tianjin 300070 (CN); FENG, Yukuan, Tianjin 300060 (CN); LIU, Xiaomeng, Tianjin 300070 (CN); XIE, Xinru, Tianjin 300070 (CN)
(74) Representative: Regimbeau
(86) International application number: PCT/CN2023/134024
(87) International publication number: WO 2024/109935

(57) **Abstract**

The present disclosure relates to a gramine-platinum(IV) complex as well as a preparation method therefor and the anti-tumor use thereof. One gramine molecule is introduced to one side of the axial direction of a platinum(IV) coordination center to form a mono-substituted tetravalent platinum complex; or one gramine molecule is introduced to one side of the axial direction of the platinum(IV) coordination center, and one molecule of a -NH-aliphatic chain group or an aliphatic chain group is introduced to the other side so as to form a bi-substituted tetravalent platinum complex. The prepared complex has stronger killing ability on tumor cells, and has obviously improved antiproliferative activity in various cell lines; and in triple-negative breast cancer cells having an extremely high malignant degree, the complex compared with clinical platinum drugs has most remarkable effect improvement. The gramine-platinum(IV) complex of the present disclosure has a simple synthesis process and low cost; said complex greatly improves the curative effect of combined drug therapy; and compared with traditional divalent platinum and similar tetravalent platinum drugs, said complex has the advantages of good curative effect, small side effect and the like.

## Description

### TECHNICAL FIELD

The present disclosure belongs to the field of anti-cancer chemical drug technology, and particularly relates to a gramine-platinum(IV) complex and preparation method and anti-tumor use thereof.

### BACKGROUND

Cancer is a major disease threatening human life and health worldwide. The incidence rate of cancer is increasing year by year, and the mortality rate thereof is very high. The International Agency for Research on Cancer (IARC) of the World Health Organization has released the latest global cancer burden statistics 2020 including the incidence and mortality rates of 36 cancers in 185 countries, as well as the development tendency of cancer. In 2020, the global number of new cancer cases was estimated to be 19.3 million, and cancer deaths reached nearly 10 million. The top ten cancers in terms of the incidence rate in the world were breast cancer with 2.26 million cases (11.7%), lung cancer with 2.21 million cases (11.4%), colorectal cancer with 1.88 million cases (9.8%), prostate cancer with 1.41 million cases (7.3%), skin cancer with 1.20 million cases (6.2%), gastric cancer with 1.09 million cases (5.6%), liver cancer with 0.91 million cases (4.7%), cervical cancer with 0.6 million cases (3.1%), esophageal cancer with 0.6 million cases (3.1%), and thyroid cancer with 0.59 million cases (3%), respectively. These ten cancers accounted for 63% of the total number of newly diagnosed cancers.

Chemotherapy and radiotherapy are the main methods for treating cancers. Chemotherapy is irreplaceable in the treatment of tumors, and therefore, the development of chemotherapy drugs is still a research focus. As is well-known, platinum drugs such as cisplatin, oxaliplatin and carboplatin are important drugs for clinical treatment of tumors, mainly for treating breast cancer, bladder cancer, lung cancer, malignant lymphoma, etc. The platinum drug attacks DNA as the main target, and exhibits a significant anti-cancer effect with strong anti-cancer activity. The cross resistance is rarely developed between the platinum drug and other anti-cancer drugs, which is beneficial for clinical combined administration. The platinum drug has a broad-spectrum anti-cancer effect. However, the toxic side effect and drug resistance of the platinum drug limit its clinical application, and the seeking of new platinum drugs has become a research focus. Pt(IV) complexes have wide application prospect due to their diverse activities and low systemic toxicity.

Pt(IV) complex is a product obtained by oxidation of cisplatin, oxaliplatin and the like. Compared with divalent platinum, Pt(IV) complex has an octahedral configuration and is pharmacokinetically inert, which improves its stability in blood and prolongs the action time of platinum drugs, thereby enhancing anti-tumor activity. Moreover, Pt(IV) complex has high lipophilicity and is easily absorbed by cancer cells. In addition, different functional molecules can be introduced into the two axial sites of Pt(IV) complex through chemical modification, so as to increase the selectivity of Pt(IV) complex towards cancer cells, to improve the pharmacological properties of Pt(IV) complex, and thus to achieve targeting and synergistic sensitization effects, and the like.

Natural products play an important role in drug development and research due to their special advantages including low toxicity, immunity, cardiovascular protection, etc. Among them, gramine (GM) is a natural indole alkaloid with various pharmacological effects such as anti-inflammatory, anti-tumor, anti-bacterial and anti-oxidant properties. Gramine can exert anti-tumor effects by regulating various signaling pathways. Gramine can significantly inhibit tumor growth and metastasis by inhibiting transforming growth factor-beta (TGF-β), and the mechanism thereof involves blocking cell cycle, promoting cell apoptosis, inhibiting tumor angiogenesis, inhibiting EMT occurrence, inhibiting tumor invasion and metastasis, *etc.* Therefore, the present disclosure is intended to introduce gramine as a functional ligand into an axial position of tetravalent platinum to synthesize a gramine-platinum (IV) prodrug which enables to achieve the synergistic sensitization effects.

### SUMMARY

In order to solve the above technical problem, the present disclosure provides a gramine-platinum (IV) complex and preparation method and anti-tumor use thereof.

A technical solution of the present disclosure is a gramine-platinum(IV) complex in which a gramine group is attached to a side in the axial direction of the tetravalent platinum coordination center, or a gramine group is attached to one side in the axial direction of the tetravalent platinum coordination center while a -NH-aliphatic chain group or an aliphatic chain group is attached to the other side in the axial direction of the tetravalent platinum coordination center.

In some embodiments, the gramine-platinum(IV) complex has a structure as shown in formula 1; 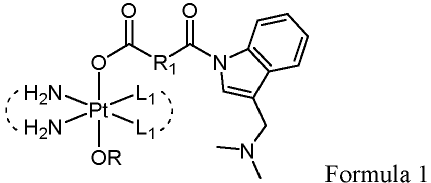
wherein, R is hydrogen atom, -C(O)-R₂, deuterium atom or C₁₋₆ alkyl; 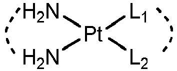
is selected from the group consisting of cisplatin, oxaliplatin, carboplatin, heptaplatin, nedaplatin, lobaplatin and miriplatin,
preferably, 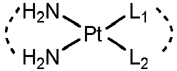 is cisplatin, oxaliplatin or carboplatin;
R₁ is -CₙH₂ₙ-, in which n is an integer and 1 ≤ n ≤ 6, and preferably, -CₙH₂ₙ- is a linear chain group;
R₂ is -CₘH₂ₘ₊₁ or -NH-CₘH₂ₘ₊₁, in which m is an integer and 1 ≤ m ≤ 20, and preferably, -CₘH₂ₘ₊₁ or -NH-CₘH₂ₘ₊₁ is a linear chain group.

In some embodiments, the gramine-platinum(IV) complex has a structure as shown in formula 1-1 or 1-2; 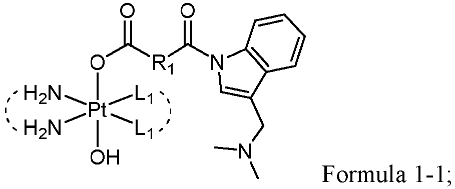 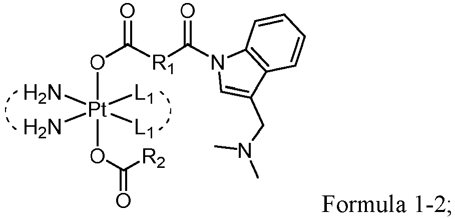 wherein, 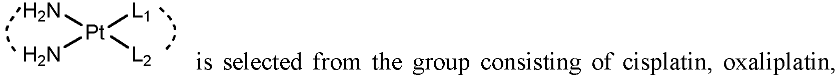 carboplatin, heptaplatin, nedaplatin, lobaplatin and miriplatin,
R₁ is -CₙH₂ₙ-, in which n is an integer and 1 ≤ n ≤ 6;
R₂ is -CₘH₂ₘ₊₁ or -NH-CₘH₂ₘ₊₁, in which m is an integer and 1 ≤ m ≤ 20.

In some embodiments, provided is the gramine-platinum(IV) complex in which 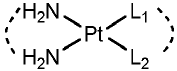 is cisplatin, oxaliplatin or carboplatin.

In some embodiments, provided is the gramine-platinum(IV) complex in which - CₙH₂ₙ- is a linear chain group and n is 1 or 2.

In some embodiments, provided is the gramine-platinum(IV) complex in which - CₘH₂ₘ₊₁ or -NH-CₘH₂ₘ₊₁ is a linear chain group.

In some embodiments, provided is the gramine-platinum(IV) complex in which - CₙH₂ₙ- is a linear chain group.

In some embodiments, provided is the gramine-platinum(IV) complex in which - CₘH₂ₘ₊₁ or -NH-CₘH₂ₘ₊₁ is a linear chain group with 6 ≤ m ≤ 17, preferably 6 ≤ m ≤ 15, and more preferably m being 6, 8, 12 or 15.

In some embodiments, the gramine-platinum(IV) complex has a structure as shown in any one of formulas 2 to 10:

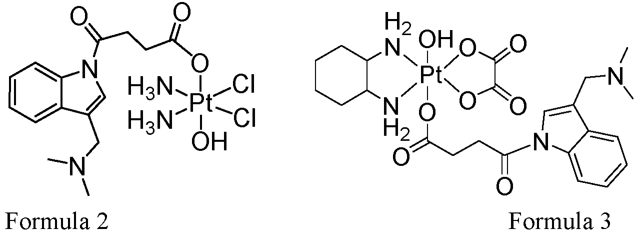

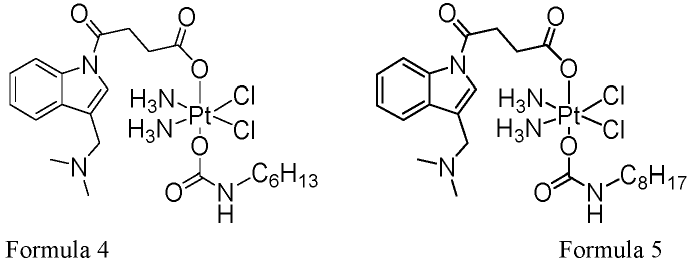

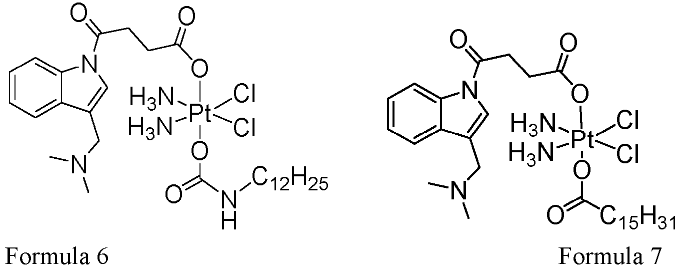

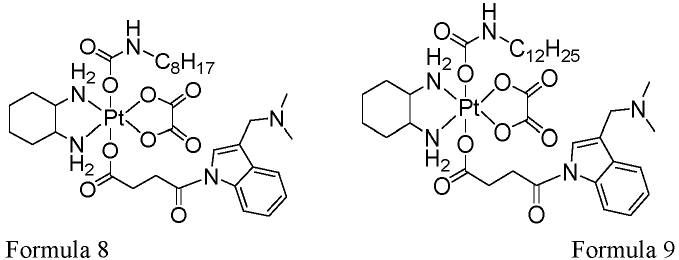

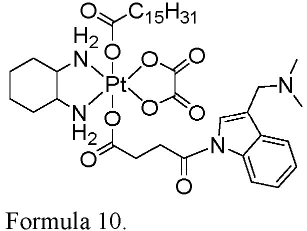

The present disclosure also provides a method for preparing the gramine-platinum(IV) complex, comprising: subjecting a compound of formula 11 and a compound of formula 17 to the esterification reaction in the presence of a first condensing agent and a first acid binding agent to obtain the compound of formula 1; 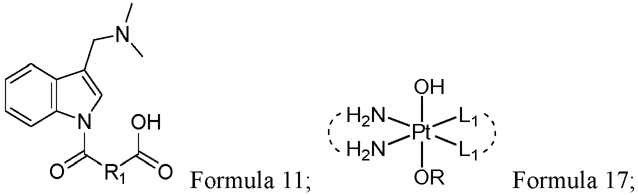
in particular, subjecting the compound of formula 11 and a compound of formula 12 are subjected to the esterification reaction in the presence of the first condensing agent and the first acid binding agent to obtain the compound of formula 1-1; 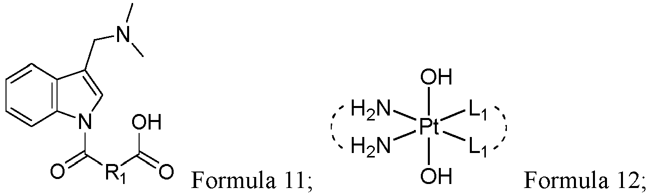
wherein,
R is hydrogen atom, -C(O)-R₂, deuterium atom or C₁₋₆ alkyl; 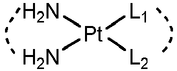
is selected from the group consisting of cisplatin, oxaliplatin, carboplatin, heptaplatin, nedaplatin, lobaplatin and miriplatin, and R₁ is -CₙH₂ₙ- in which n is an integer and 1 ≤ n ≤ 6.

In some embodiments, provided is the method for preparing the gramine-platinum(IV) complex, wherein 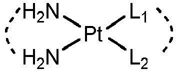 is cisplatin, oxaliplatin or carboplatin.

In some embodiments, -CₙH₂ₙ- in the gramine-platinum(IV) complex is a linear chain group.

In some embodiments, provided is the method for preparing the gramine-platinum(IV) complex, wherein the first condensing agent is TBTU; the first acid binding agent is triethylamine; the ratio of raw materials in the reaction is as follows: the compound of formula 11: the compound of formula 17 or the compound of formula 12: the first condensing agent: the first acid binding agent is 1 to 1.2 : 1 : 1.2 to 2 : 1.2 to 2.

In some embodiments, the reaction is carried out under the dark condition with inert gas protection.

In some embodiments, provided is the method for preparing the gramine-platinum(IV) complex, comprising: subjecting the compound of formula 1-1 and a compound of formula 13 or a compound of formula 14 to the esterification reaction to obtain the compound of formula 1-2; 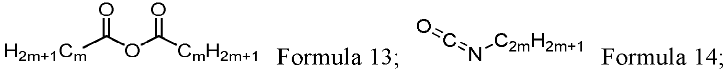 wherein,
m is an integer and 1 ≤ m ≤ 20.

In some embodiments, -CₘH₂ₘ₊₁ in the gramine-platinum(IV) complex is a linear chain group.

In some embodiments, provided is the method for preparing the gramine-platinum(IV) complex, comprising subjecting gramine of formula 15 and a compound of formula 16 to the acylation reaction in the presence of a second condensing agent and a second acid binding agent to obtain the compound of formula 11;

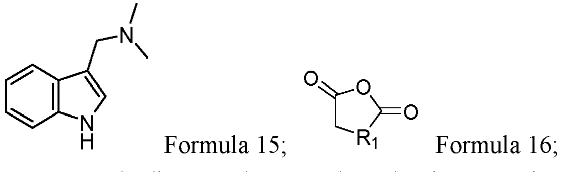

**In** some embodiments, the second condensing agent is DMAP; and the second acid binding agent is triethylamine.

The present disclosure also provides an isotopically substituted form of the above gramine-platinum(IV) complex. In some embodiments, the isotopically substituted form is a deuterated form.

The present disclosure also provides a pharmaceutical composition comprising a therapeutically effective amount of at least one of the gramine-platinum(IV) complex above or an isotopically substituted form thereof, and a pharmaceutically acceptable excipient.

In some embodiments, the unit dose of the pharmaceutical composition is 0.001 mg to 1000 mg.

In some embodiments, the pharmaceutical composition comprises 0.01% to 99.99% of the gramine-platinum(IV) complex above or an isotopically substituted form thereof, based on the total weight of the composition. In some embodiments, the pharmaceutical composition comprises 0.1% to 99.9% of the gramine-platinum(IV) complex above or an isotopically substituted form thereof. In some embodiments, the pharmaceutical composition comprises 0.5% to 99.5% of the gramine-platinum(IV) complex above or an isotopically substituted form thereof. In some embodiments, the pharmaceutical composition comprises 1% to 99% of the gramine-platinum(IV) complex above or an isotopically substituted form thereof. In some embodiments, the pharmaceutical composition comprises 2% to 98% of the gramine-platinum(IV) complex above or an isotopically substituted form thereof.

In some embodiments, the pharmaceutical composition comprises 0.01% to 99.99% of a pharmaceutically acceptable excipient, based on the total weight of the composition. In some embodiments, the pharmaceutical composition comprises 0.1% to 99.9% of a pharmaceutically acceptable excipient. In some embodiments, the pharmaceutical composition comprises 0.5% to 99.5% of a pharmaceutically acceptable excipient. In some embodiments, the pharmaceutical composition comprises 1% to 99% of a pharmaceutically acceptable excipient. In some embodiments, the pharmaceutical composition comprises 2% to 98% of a pharmaceutically acceptable excipient.

In another aspect, the present disclosure also provides a compound of formula 11 or a pharmaceutically acceptable salt thereof, 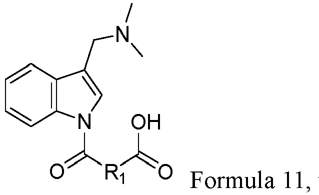 wherein R₁ is -CₙH₂ₙ- in which n is an integer and 1 ≤ n ≤ 6.

In some embodiments, -CₙH₂ₙ- in the compound of formula 11 is a linear chain group.

In some embodiments, the compound of formula 11 is selected from the group consisting

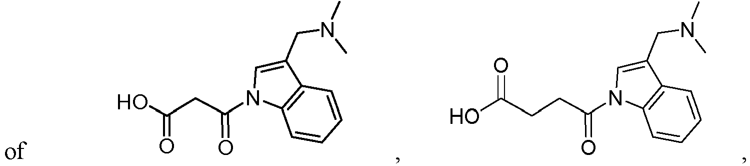

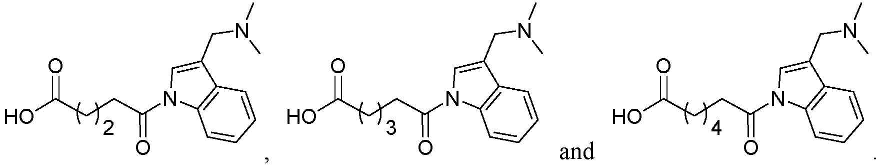

The present disclosure also provides a use of the gramine-platinum(IV) complex above or the isotopically substituted form thereof or the compound of formula 11 or the pharmaceutically acceptable salt thereof or the pharmaceutical composition above in the preparation of an anti-tumor medicament.

In some embodiments, the tumor is selected from the group consisting of breast cancer (such as triple negative breast cancer), colorectal cancer, cervical cancer, head and neck cancer (such as pharyngeal squamous carcinoma), bladder cancer, ovarian cancer, pancreatic cancer, liver cancer and lung cancer.

In another aspect, the present disclosure also provides a method for treating/preventing a tumor, comprising administering a therapeutically effective amount of the gramine-platinum(IV) complex or the isotopically substituted form thereof or the compound of formula 11 or the pharmaceutically acceptable salt thereof or the pharmaceutical composition above, to an individual. In some embodiments, the tumor includes, but is not limited to breast cancer (such as triple negative breast cancer), colorectal cancer, cervical cancer, pharyngeal squamous carcinoma, bladder cancer, ovarian cancer, pancreatic cancer, liver cancer and lung cancer.

The present disclosure also provides the gramine-platinum(IV) complex or the isotopically substituted form thereof or the compound of formula 11 or the pharmaceutically acceptable salt thereof or the pharmaceutical composition above, for use in the treatment/prevention of a tumor.

In some embodiments, the tumor includes, but is not limited to breast cancer (such as triple negative breast cancer), colorectal cancer, cervical cancer, head and neck cancer (such as pharyngeal squamous carcinoma), bladder cancer, ovarian cancer, pancreatic cancer, liver cancer and lung cancer.

In another aspect, the present disclosure also provides a use of the gramine-platinum(IV) complex above or the isotopically substituted form thereof in combination with an immunosuppressant in the preparation of a medicament for treating a tumor.

In some embodiments, the tumor includes, but is not limited to breast cancer (such as triple negative breast cancer), colorectal cancer, cervical cancer, head and neck cancer (such as pharyngeal squamous carcinoma), bladder cancer, ovarian cancer, pancreatic cancer, liver cancer and lung cancer.

In some embodiments, the immunosuppressant is selected from the group consisting of an anti-PD-1 antibody or an antigen-binding fragment thereof, an anti-PD-L1 antibody or an antigen-binding fragment thereof, and an anti-CTLA-4 antibody or an antigen-binding fragment thereof.

In some embodiments, the anti-PD-1 antibody or the antigen-binding fragment thereof includes, but is not limited to Pembrolizumab, Nivolumab, Camrelizumab, Toripalimab, Tislelizuma, Cemiplimab, Sintilimab, CS-1003, HLX-10, SCT-I10A, sasanlimab, spartalizumab, MGD-013, retifanlimab, MEDI-0680, BAT-1306, MEDI-5752, LZM-009, JTX-4014, BI-754091, AK-104, BCD-217, balstilimab, AK-103 or cetrelimab. In some embodiments, the anti-PD-1 antibody or the antigen-binding fragment thereof is Camrelizumab. In some embodiments, the anti-PD-1 antibody or the antigen-binding fragment thereof is Toripalimab. In some embodiments, the anti-PD-1 antibody or the antigen-binding fragment thereof is Tislelizuma. In some embodiments, the anti-PD-1 antibody or the antigen-binding fragment thereof is Cemiplimab.

In some embodiments, the anti-PD-L1 antibody or the antigen-binding fragment thereof is selected from the group consisting of Atezolizumab, Avelumab, Durvalumab, Adebrelimab, Envafolimab, CS-1001, TQB-2450, KL-A167, IMC-001 and CX-072. In some embodiments, the anti-PD-L1 antibody or the antigen-binding fragment thereof is Adebrelimab.

In some embodiments, the anti-PD-L1 antibody or the antigen-binding fragment thereof is Ipilimumab or Tremelimumab.

In another some embodiments, the dose of the immunosuppressant is 0.1 to 10.0 mg/kg, and may be 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg.

The present disclosure also provides the gramine-platinum(IV) complex or the isotopically substituted form thereof for use in the treatment of a tumor in combination with an immunosuppressant.

The present disclosure also provides an immunosuppressant for use in the treatment of a tumor in combination with the gramine-platinum(IV) complex or the isotopically substituted form thereof.

The present disclosure also provides a composition comprising an immunosuppressant, and the gramine-platinum(IV) complex above or the isotopically substituted form thereof, and a pharmaceutically acceptable excipient.

The present disclosure also provides a method for treating a tumor, comprising: administering an effective dose of the gramine-platinum(IV) complex above or the isotopically substituted form thereof, and an immunosuppressant, to a patient.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. There may be additional asymmetric carbon atoms in substituents such as alkyl. All these isomers and mixtures thereof fall within the scope of the present disclosure. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structure of the compound of the present disclosure, the bond " 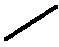 " represents an unspecified configuration, and that is, if chiral isomers exist in the chemical structure, the bond " 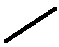 " may be " 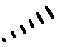 " or " 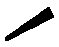 ", or contains both the configurations of " 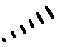 " and " 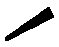 ". In addition, the bond " 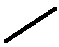 " represents not only a covalent bond within the molecule, but also an ionic bond or a coordination bond. For example, cisplatin can be represented by

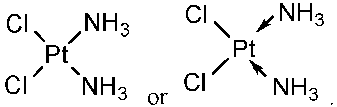

The present disclosure further includes isotopically-labeled compounds which are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

Unless otherwise specified, when a position is specifically assigned to deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is at least 1000 times, at least 2000 times, at least 3000 times, at least 4000 times, at least 5000 times, at least 6000 times, or higher times greater than the natural abundance of deuterium. The present disclosure further includes various deuterated forms of the compound of formula (I). Each available hydrogen atom connected to a carbon atom may be independently replaced by a deuterium atom. Those skilled in the art are able to synthesize the deuterated forms of the compound of formula (I) with reference to relevant literatures. In preparing the deuterated forms of the compound of formula (I), commercially available deuterated starting materials can be used, or they can be synthesized using conventional techniques with deuterated reagents including, but not limited to deuterated borane, tri-deuterated borane in tetrahydrofuran solution, deuterated lithium aluminum hydride, deuterated iodoethane, deuterated iodomethane and the like.

The term "fatty chain group" or "aliphatic group" refers to a group composed of carbon and hydrogen, usually with a linear or branched chain structure.

The term "C₁₋₆ alkyl" refers to a saturated linear or branched aliphatic hydrocarbon group having 1 to 6 (e.g., 1, 2, 3, 4, 5 or 6) carbon atoms. Non-limiting examples include methyl, ethyl, n-propyl, isopropyl, n-butyl, isobutyl, tert-butyl, sec-butyl, n-pentyl, n-hexyl, etc.

The term "pharmaceutical composition" represents a mixture containing one or more compounds described herein or physiologically/pharmaceutically acceptable salts or prodrugs thereof, and other chemical components, and other components, for example, physiologically/pharmaceutically acceptable carriers and excipients. The pharmaceutical composition is intended to promote administration to an organism and facilitate the absorption of the active ingredient so that it can exert its biological activity.

The "effective amount" in the present disclosure includes an amount sufficient to allow or facilitate diagnosis. The effective amount can be the maximum dose or administration regimen that avoids significant side effects or toxic effects.

The term "pharmaceutically acceptable excipient" includes, but is not limited to, any adjuvant, carrier, glidant, sweetener, diluent, preservative, dye/colorant, flavor enhancer, surfactant, wetting agent, dispersant, suspending agent, stabilizer, isotonic agent, solvent or emulsifier that has been approved by the U.S. Food and Drug Administration and is acceptable for use in humans or livestock animals.

The term "antibody" encompasses a variety of antibody structures, including, but not limited to monoclonal antibodies, polyclonal antibodies, monospecific antibodies, multispecific antibodies (e.g., bispecific antibodies), full-length antibodies and antibody fragments (or antigen-binding fragments, or antigen-binding moieties) so long as they exhibit the desired antigen-binding activity. An antibody may refer to an immunoglobulin represented as a four-peptide-chain structure which is formed by linking two identical heavy chains and two identical light chains by interchain disulfide bonds. The heavy chain constant regions of immunoglobulins differ in their amino acid compositions and arrangements, and thus in their antigenicity. Accordingly, immunoglobulins can be divided into five classes, or described as immunoglobulin isotypes, namely IgM, IgD, IgG, IgA, and IgE, with their corresponding heavy chains being µ chain, δ chain, γ chain, α chain, and ε chain, respectively. Additionally, Ig of the same class can be divided into different subclasses according to differences in the amino acid compositions of the hinge regions and the numbers and positions of disulfide bonds of the heavy chains, and for example, IgG can be divided into IgG1, IgG2, IgG3, and IgG4. Light chains are divided into κ or λ chains according to differences in the constant regions. Each of the five classes of Ig may have a κ or λ chain. In the antibody heavy and light chains, the sequences of about 110 amino acids near the N-terminus vary considerably and thus are referred to as variable regions (V regions); and the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

The antibody of the present disclosure may be polyclonal, monoclonal, xenogenic, allogeneic, syngeneic, or modified forms thereof, with the monoclonal antibody being particularly useful in various embodiments. Generally, the antibody of the present disclosure is a recombinant antibody. The term "recombinant" used herein generally refers to products such as a cell, polynucleotide, protein or vector, and indicates that the cell, polynucleotide, protein or vector has been modified by the introduction of a heterologous polynucleotide or protein or the alteration of a native polynucleotide or protein, or the cell is derived from a cell so modified. For example, recombinant cells express genes that are not found within the native (non-recombinant) cellular form, or express native genes that are abnormally expressed, lowly expressed or not expressed at all.

The term "antigen-binding fragment" encompasses a single-chain antibody (i.e., full-length heavy and light chains); Fab, a modified Fab, Fab', a modified Fab', F(ab')2, Fv, Fab-Fv, Fab-dsFv, a single-domain antibody (e.g., VH or VL or VHH), scFv, a bivalent or trivalent or tetravalent antibody, Bis-scFv, a diabody, a tribody, a triabody, a tetrabody, and an epitope-binding fragment of any one of the above (see, e.g., Holliger and Hudson, 2005, Nature Biotech. 23 (9):1126-1136; Adair and Lawson, 2005, Drug Design Reviews-Online 2 (3), 209-217). Methods for producing and preparing these antigen-binding fragments are well-known in the art (see, e.g., Verma et al., 1998, Journal of Immunological Methods, 216, 165-181).

For the determination or definition of CDRs, the deterministic depiction of CDRs and identification of residues comprising binding sites of the antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including, but not limited to the Kabat numbering scheme, the Chothia numbering scheme, the AbM numbering scheme, the IMGT numbering scheme, the contact definition, and the conformational definition.

The present disclosure has the advantages and positive effects of combining gramine with a platinum drug to synthesize a new gramine-platinum (IV) complex which exhibits better biological activity than the parent cisplatin, has an IC₅₀ value lower than cisplatin by tens of times, and has good anti-proliferation ability against a variety of cancer cells, especially against triple negative breast cancer cells with extremely high malignancy, showing the most remarkably improved effect than the clinical platinum drug. In addition, the introduction of long aliphatic chain increases the stability of drugs, while enhancing the lipid solubility and transmembrane ability of Pt (IV) molecules, thereby promoting the absorption of platinum drugs. The gramine-platinum (IV) complex with double substitution of gramine and aliphatic chain exhibits significantly improved drug stability and cellular uptake, and not only enhances anti-tumor activity, but also has the effects of attenuation and sustained release. The synthesis process of the prodrug is simple and low-cost. The gramine-platinum (IV) complex greatly improves the efficacy of combined administration, and compared with traditional divalent platinum and similar tetravalent platinum drugs, it has the advantages of good efficacy, minimal side effect, etc, and provides a new idea for the modification of tetravalent platinum.

### BRIEF DESCRIPTION OF THE DRAWINGS

Figure 1 shows the intracellular release of Compound c in Example 11.
Figure 2 shows the change in body weight of mice in different administration groups in Example 12 (** P<0.01, *** P<0.001).
Figure 3 shows the change in tumor volume of mice in different administration groups in Example 12 (** P<0.01, *** P<0.001).
Figure 4 shows the final tumor weight of mice in Example 12 (** P<0.01, *** P<0.001).
Figure 5 shows the survival rate of mice in different administration groups in Example 12.
Figure 6 shows the final distribution of platinum (Pt) in various tissues in Example 12.
Figure 7 shows the final image of tumors in Example 12.
Figure 8 shows the H&E staining results of mouse organs and tumor tissues in Example 12.
Figure 9 shows the immunohistochemical staining results of mouse spleens in Example 12.
Figure 10 shows the curves of tumor volume change of mice in the PDX model of pancreatic cancer (** P<0.01, *** P<0.001).
Figure 11 shows the tumor weight of mice in the PDX model of pancreatic cancer (** P<0.01, *** P<0.001).
Figure 12 shows the curves of body weight change of mice in the PDX model of pancreatic cancer (** P<0.01, *** P<0.001).
Figure 13 shows the distribution of platinum (Pt) in various tissues in the PDX model of pancreatic cancer (** P<0.01, *** P<0.001).
Figure 14 shows the curves of volume change of mice in the KPC model of pancreatic cancer (** P<0.01, *** P<0.001).
Figure 15 shows the tumor weight of mice in the KPC model of pancreatic cancer (** P<0.01, *** P<0.001).
Figure 16 shows the curves of body weight change of mice in the KPC model of pancreatic cancer (** P<0.01, *** P<0.001).

### DETAILED DESCRIPTION

The present disclosure discloses a gramine-platinum(IV) complex in which one molecule of gramine is connected to a side in the axial direction of the tetravalent platinum coordination center to form a monosubstituted tetravalent platinum complex with a structure as shown in formula 1-1; 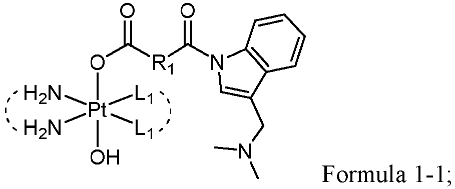 wherein, 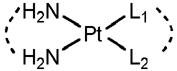 is selected from the group consisting of cisplatin, oxaliplatin, carboplatin, heptaplatin, nedaplatin, lobaplatin and miriplatin, and for example, 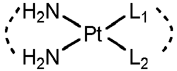 is cisplatin, oxaliplatin or carboplatin; R₁ is -CₙH₂ₙ- in which n is an integer and n≥1 and for example n≤6 or n≤2, with a better effect when - CₙH₂ₙ- is a linear chain group.

Gramine is combined with a platinum drug to synthesize a new gramine-platinum(IV) complex. Gramine is a natural indole alkaloid with various pharmacological effects such as anti-inflammatory, anti-tumor, anti-bacterial and anti-oxidant properties. Gramine can exert anti-tumor effects by regulating various signaling pathways. Gramine can significantly inhibit tumor growth and metastasis by inhibiting TGF-β signaling pathway, and the mechanism thereof involves blocking cell cycle, promoting cell apoptosis, inhibiting tumor angiogenesis, inhibiting EMT occurrence, inhibiting tumor invasion and metastasis, etc. In some embodiments of the present disclosure, the gramine-platinum(IV) complex can be represented by structural formula 2 or 3:

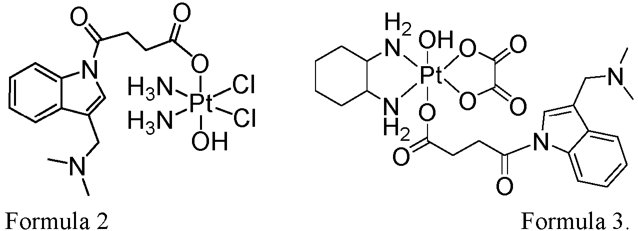

The gramine-platinum(IV) complex of structural formula 1-1 is prepared by using the following method:
Step 1: subjecting gramine of formula 15 and a compound of formula 16 to the acylation reaction in the presence of a second condensing agent and a second acid binding agent to obtain a compound of formula 11; 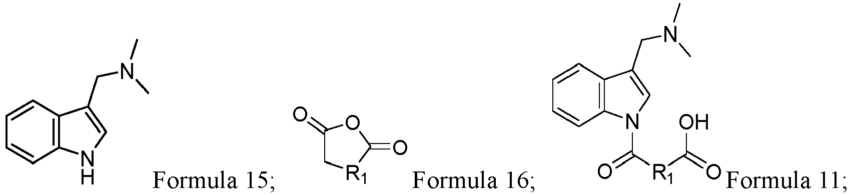 wherein, the second condensing agent is DMAP; and the second acid binding agent is triethylamine;
Step 2: subjecting the compound of formula 11 and a compound of formula 12 to the esterification reaction in the presence of a first condensing agent and a first acid binding agent to obtain the compound of formula 1-1; 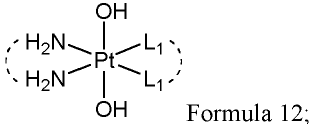 wherein, the first condensing agent is TBTU; the first acid binding agent is triethylamine; the ratio of raw materials in the reaction is as follows: the compound of formula 11: the compound of formula 12: the first condensing agent: the first acid binding agent is 1 to 1.2 : 1 : 1.2 to 2 : 1.2 to 2; and the reaction is carried out under the dark condition with inert gas protection.

The gramine-platinum(IV) complex of the present disclosure is a prodrug forms which can release the gramine ligand and parent platinum drug in cells, aiming to reduce the toxic side effect of the platinum drug while achieving the synergistic sensitization effects. In order to improve the uptake and stability of Pt(IV), gramine is attached to one side in the axial direction of the tetravalent platinum coordination center, and aliphatic chains with different lengths are introduced at the hydroxy group on the other axial side of platinum. The structure is shown in formula 1-2. 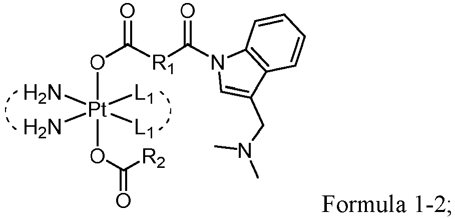 wherein, 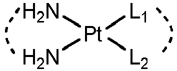 is selected from the group consisting of cisplatin, oxaliplatin, carboplatin, heptaplatin, nedaplatin, lobaplatin and miriplatin, and for example, 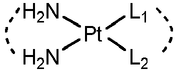 is cisplatin, oxaliplatin or carboplatin; R₁ is -CₙH₂ₙ- in which n is an integer and n≥1 and for example n≤6 or n≤2, with a better effect when - CₙH₂ₙ- is a linear chain group; and R₂ is -CₘH₂ₘ₊₁ or -NH-CₘH₂ₘ₊₁ in which m≥1 and for example m≤20 or m≤17. In some embodiments, provided is the gramine-platinum(IV) complex in which 2≤m≤17, with a better effect when -CₘH₂ₘ₊₁ or -NH-CₘH₂ₘ₊₁ is a linear chain group.

The introduction of long aliphatic chain increases the stability of drugs, while enhancing the lipid solubility and transmembrane ability of Pt(IV) molecules, thereby promoting the absorption of platinum drugs. The gramine-platinum(IV) complex with double substitution of gramine and aliphatic chain exhibits significantly improved drug stability and cellular uptake, and not only enhances anti-tumor activity, but also has the effects of attenuation and sustained release. The synthesis process of the prodrug is simple and low-cost. The gramine-platinum(IV) complex greatly improves the efficacy of combined administration, and compared with traditional divalent platinum and similar tetravalent platinum drugs, it has the advantages of good efficacy, minimal side effect, etc.

In some embodiments of the present disclosure, the gramine-platinum(IV) complex can be represented by any one of structural formulas 4 to 10:

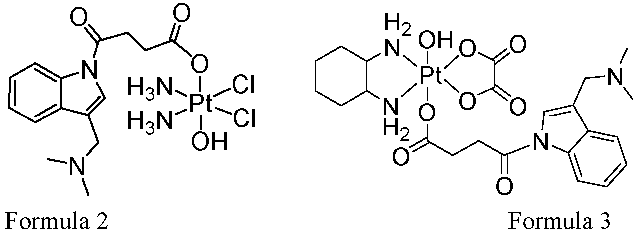

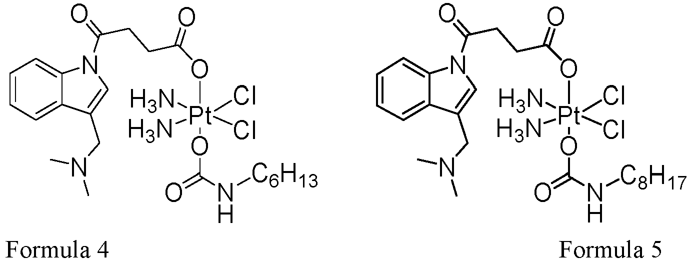

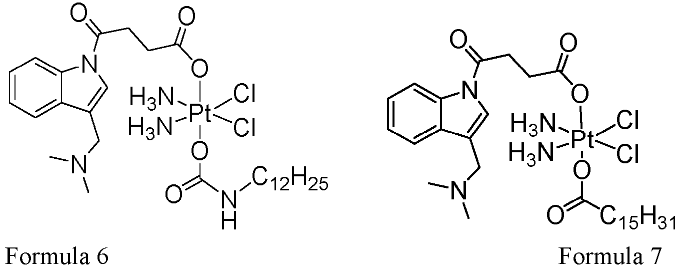

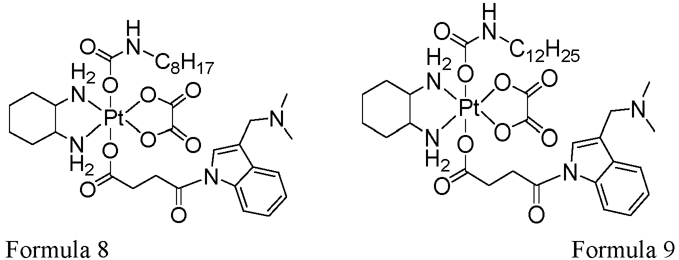

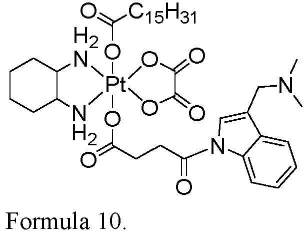

The gramine-platinum (IV) complex of structural formula 1-2 is prepared by using the following method:
subjecting the compound of formula 1-1 and a compound of formula 13 or a compound of formula 14 to the esterification reaction to obtain the gramine-platinum (IV) complex of formula 1-2;

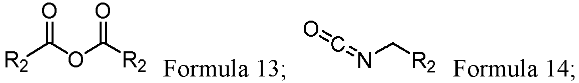

The prepared gramine-platinum(IV) complex above can be used for the preparation of anti-tumor drugs, such as drugs against breast cancer (triple negative breast cancer), colorectal cancer, cervical cancer, head and neck cancer (such as pharyngeal squamous carcinoma), bladder cancer, ovarian cancer, pancreatic cancer, liver cancer and lung cancer. The gramine-platinum(IV) complex exhibits better biological activity than the parent cisplatin, has an IC₅₀ value lower than cisplatin by tens of times, and has good anti-proliferation ability against a variety of cancer cells, especially against triple negative breast cancer cells with extremely high malignancy, showing the most remarkably improved effect than the clinical platinum drug.

Hereinafter, the technical solutions of the present disclosure will be illustrated in conjunction with examples and drawings. However, the scope of the present disclosure is not limited by these examples. The experimental methods without specifying specific operating steps shall be performed according to the corresponding product instruction. Unless otherwise specified, the instruments, reagents and consumables used in the examples can be purchased from commercial companies.

### Example 1:

The gramine-platinum(IV) complex **a3** of the present example has the following structural formula:

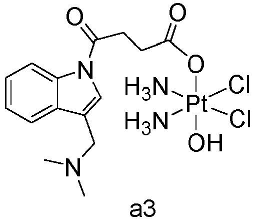

The preparation method (synthetic route) of the gramine-platinum(IV) complex of the present example was as follows:

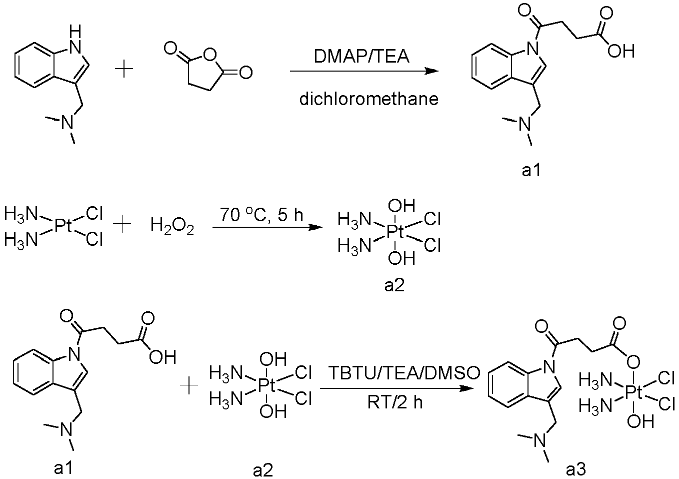

Step 1: Gramine (2 g, 11.5 mmol) was weighed exactly and put in a 50 mL round-bottom flask and 20 mL of dichloromethane was added. Under stirring, succinic anhydride (2.30 g, 23 mmol), 4-dimethylaminopyridine DMAP (0.14 g, 1.15 mmol), and triethylamine (3.18 mL, 23 mmol) were added successively, and the mixture was left to react overnight at room temperature. After the completion of the reaction monitored by TLC, the solvent was removed by rotary evaporation, and recrystallization was carried out with methanol to obtain a white solid, which was dried under vacuum to obtain Compound **a1** (2.74 g, yield 86.9%).

Step 2: Cisplatin (1.00 g, 3.33 mmol) was weighed exactly and put in a 25 mL round-bottom flask, and 2 mL of distilled water was added. After stirring, H₂O₂ (30% w/v, 10.0 mL) was slowly added dropwise and the mixture was left to react at 70° C in the dark for 5 hours. After the completion of the reaction, the reaction solution was cooled to room temperature and left overnight at 4°C. The supernatant was then removed by centrifugation to obtain a light yellow solid. The solid was washed twice with distilled water, ethanol and ether respectively, and then dried under vacuum to obtain Compound **a2** (858.60 mg, yield 77.4%).

Step 3: Compound **a1** (100 mg, 0.36 mmol) was weighed exactly and put in a 10 mL round-bottom flask, and 1 mL of ultra dry DMSO was added thereto. Under stirring, TBTU (174 mg, 0.54 mmol) and TEA (75 µL, 0.54 mmol) were added. After 15 minutes, Compound **a2** (120 mg, 0.36 mmol) was added, and the mixture was left to react overnight at room temperature under argon protection in the dark. When the reaction solution became clear, it was detected by TCL. Then, the reaction solution was poured into 20 mL of dichloromethane, sonicated, and centrifuged to collect a precipitate. The precipitate was washed with dichloromethane and methanol respectively, and dried in a vacuum drying oven to obtain Compound **a3** as a light yellow solid (91.76 mg, yield 43.2%).

¹H NMR (400 MHz, DMSO-*d*₆): δ 8.33 8.35 (d, *J* = 7.9 Hz, 1H), 8.01 (s, 1H), 7.80 (d, *J* = 7.3 Hz, 1H), 7.45 - 7.29 (m, 2H), 6.11 - 5.78 (m, 6H), 3.42 (s, 2H), 3.26 - 3.14 (m, 2H), 2.67 (t, *J* = 10.6 Hz, 2H), 2.51 (s, 6H).

¹³C NMR (101 MHz, DMSO-*d*₆): δ 179.37, 171.41, 135.08, 129.70, 125.02, 123.90, 123.38, 119.65, 118.76, 115.99, 54.90, 42.91, 31.43, 30.61.

HR-MS (m/z): calculated C₁₅H₂₄Cl₂N₄O₄Pt (M + H)⁺, 590.08223; found: 590.08990.

### Example 2:

The gramine-platinum(IV) complex a of the present example has the following structural formula:

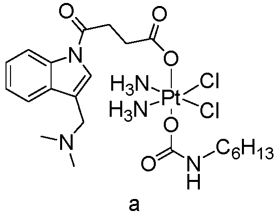

The preparation method (synthetic route) of the gramine-platinum(IV) complex of the present example was as follows:

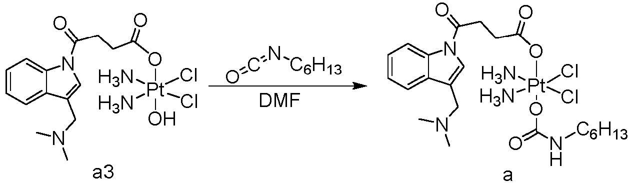

Compound **a3** (118.0 mg, 0.20 mmol) was weighed exactly and put in a 10 mL round-bottom flask, and 2 mL of ultra dry DMF was added thereto. Under magnetic stirring, hexyl isocyanate (50 µL) was added, and the mixture was left to react overnight at 60°C under argon protection in the dark. After the completion of the reaction monitored by TLC, DMF was removed by rotary evaporation in a water bath at 60°C. The residue was dissolved in 1 mL of methanol and purified by a preparative silica gel plate to obtain a white solid. The solid was washed with anhydrous ether to remove residual DMF and dried under vacuum to obtain Compound **a** (50.62 mg, yield 35.4%).

¹H NMR (400 MHz, DMSO-*d*₆): δ 8.33 (d, *J* = 8.0 Hz, 1H), 7.80 (s, 1H), 7.71 (d, *J* = 7.5 Hz, 1H), 7.32 (t, *J* = 7.6 Hz, 1H), 7.26 (t, *J* = 7.3 Hz, 1H), 6.77 (s, 6H), 6.53 (s, 1H), 3.54 (s, 2H), 3.22 - 3.14 (m, 2H), 2.88 (d, *J* = 5.5 Hz, 2H), 2.72 (t, *J* = 5.6 Hz, 2H), 2.20 (s, 6H), 1.34 (s, 2H), 1.22 (s, 6H), 0.85 (t, *J* = 6.6 Hz, 3H).

¹³C NMR (101 MHz, DMSO-*d*₆): δ 179.67, 171.18, 164.01, 135.22, 130.30, 124.75, 124.51, 122.77, 119.82, 118.50, 115.88, 53.89, 45.05, 41.04, 31.15, 31.09, 29.85, 29.69, 26.08, 21.97, 13.73.

HR-MS (m/z): calculated C₂₂H₃₇Cl₂N₅O₅Pt (M + H)⁺, 717.18195; found: 717.18939.

### Example 3:

The gramine-platinum(IV) complex **b** of the present example has the following structural formula:

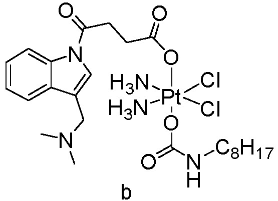

The preparation method (synthetic route) of the gramine-platinum(IV) complex of the present example was as follows:

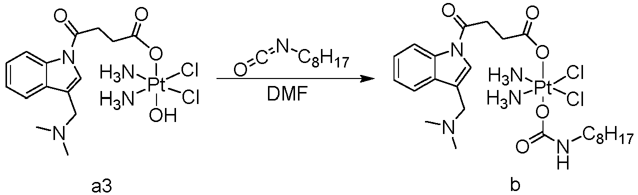

Compound **a3** (118.0 mg, 0.20 mmol) was weighed exactly and put in a 10 mL round-bottom flask, and 2 mL of ultra dry DMF was added thereto. Under magnetic stirring, octyl isocyanate (50 µL) was added, and the mixture was left to react overnight at 60°C under argon protection in the dark. After the completion of the reaction monitored by TLC, DMF was removed by rotary evaporation in a water bath at 60°C. The residue was dissolved in 1 mL of methanol and purified by a preparative silica gel plate to obtain a white solid. The solid was washed with anhydrous ether to remove residual DMF and dried under vacuum to obtain Compound **b** (54.68 mg, yield 36.7%).

¹H NMR (400 MHz, DMSO-*d*₆): δ 8.33 (d, *J* = 8.1 Hz, 1H), 7.79 (s, 1H), 7.70 (d, *J* = 7.6 Hz, 1H), 7.32 (t, *J* = 7.5 Hz, 1H), 7.26 (t, *J* = 7.3 Hz, 1H), 6.74 (s, 6H), 6.53 (s, 1H), 3.54 (s, 2H), 3.22 - 3.12 (m, 2H), 2.88 (d, *J* = 5.5 Hz, 2H), 2.72 (t, *J* = 6.1 Hz, 2H), 2.20 (s, 6H), 1.34 (s, 2H), 1.23 (s, 10H), 0.85 (d, *J* = 6.9 Hz, 3H).

¹³C NMR (101 MHz, DMSO-*d*₆): δ 179.33, 171.18, 164.02, 135.56, 130.30, 124.64, 124.48, 123.08, 119.92, 118.96, 115.98, 53.91, 45.07, 40.92, 31.30, 31.14, 29.91, 29.81, 28.78, 28.70, 26.39, 22.08, 13.94.

HR-MS (m/z): calculated C₂₄H₄₁Cl₂N₅O₅Pt (M + H)⁺, 745.21325; found: 745.22064.

### Example 4:

The gramine-platinum(IV) complex **c** of the present example has the following structural formula:

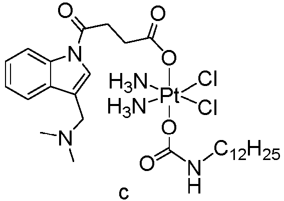

The preparation method (synthetic route) of the gramine-platinum(IV) complex of the present example was as follows:

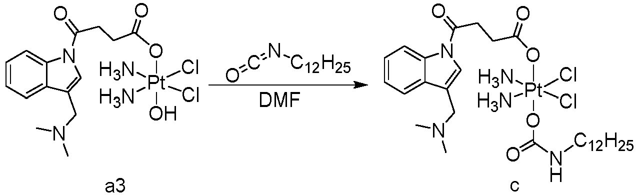

Compound **a3** (118.0 mg, 0.20 mmol) was weighed exactly and put in a 10 mL round-bottom flask, and 2 mL of ultra dry DMF was added thereto. Under magnetic stirring, dodecyl isocyanate (50 µL) was added, and the mixture was left to react overnight at 60°C under argon protection in the dark. After the completion of the reaction monitored by TLC, DMF was removed by rotary evaporation in a water bath at 60°C. The residue was dissolved in 1 mL of methanol and purified by a preparative silica gel plate to obtain a white solid. The solid was washed with anhydrous ether to remove residual DMF and dried under vacuum to obtain Compound c (49.02 mg, yield 30.6%).

¹H NMR (400 MHz, DMSO-*d*₆): δ 8.33 (d, *J* = 8.1 Hz, 1H), 7.78 (s, 1H), 7.70 (d, *J* = 7.5 Hz, 1H), 7.34 - 7.30 (m, 1H), 7.26 (t, *J* = 7.4 Hz, 1H), 6.68 (s, 6H), 6.54 (s, 1H), 3.54 (s, 2H), 3.17 (t, *J* = 6.4 Hz, 2H), 2.88 (d, *J* = 6.0 Hz, 2H), 2.73 (t, *J* = 6.3 Hz, 2H), 2.20 (s, 6H), 1.33 (s, 2H), 1.23 (s, 18H), 0.84 (d, *J* = 6.9 Hz, 3H).

¹³C NMR (101 MHz, DMSO-*d*₆): δ 179.39, 171.18, 163.76, 135.47, 130.13, 124.73, 124.55, 123.04, 120.00, 118.75, 116.04, 53.87, 45.08, 41.02, 31.24, 31.08, 31.05, 29.82, 29.06, 29.03, 29.00, 28.88, 28.60, 26.44, 26.39, 22.07, 13.94.

HR-MS (m/z): calculated C₂₈H₄₉Cl₂N₅O₅Pt (M + H)⁺, 801.27585; found: 801.28473.

### Example 5:

The gramine-platinum(IV) complex **d** of the present example has the following structural formula:

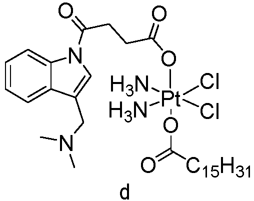

The preparation method (synthetic route) of the gramine-platinum(IV) complex of the present example was as follows:

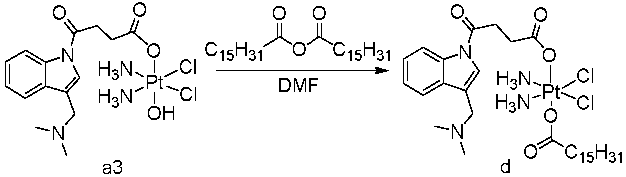

Compound **a3** (118.0 mg, 0.20 mmol) was weighed exactly and put in a 10 mL round-bottom flask, and 2 mL of ultra dry DMF was added thereto. Under magnetic stirring, palmitic anhydride (200.1 mg, 0.40 mmol) was added, and the mixture was left to react overnight at 60°C under argon protection in the dark. After the completion of the reaction monitored by TLC, DMF was removed by rotary evaporation in a water bath at 60°C. The residue was dissolved in 1 mL of methanol and purified by a preparative silica gel plate to obtain a white solid. The solid was washed with anhydrous ether to remove residual DMF and dried under vacuum to obtain Compound **d** (55.53 mg, yield 33.6%).

¹H NMR (400 MHz, DMSO-*d*₆): δ 8.33 (d, *J* = 8.1 Hz, 1H), 7.81 (s, 1H), 7.72 (d, *J* = 7.3 Hz, 1H), 7.29 (dt, *J* = 15.9, 7.3 Hz, 2H), 6.54 (s, 6H), 3.60 (s, 2H), 3.17 (t, *J* = 6.6 Hz, 2H), 2.74 (t, *J* = 6.5 Hz, 2H), 2.24 (s, 6H), 1.33 (d, *J* = 81.8 Hz, 28H), 0.85 (s, 2H).

¹³C NMR (101 MHz, DMSO-*d*₆): δ 180.91, 179.51, 171.06, 135.47, 130.12, 124.78, 124.72, 123.08, 119.92, 118.30, 115.87, 53.66, 44.85, 35.59, 31.22, 31.05, 30.99, 29.84, 29.09, 29.03, 28.99, 28.96, 28.90, 28.76, 28.68, 28.61, 25.46, 22.04, 21.09, 13.93.

HR-MS(m/z): calculated C₃₁H₅₅Cl₂N₄O₅Pt (M + H)⁺ 828.31918; found: 828.31964.

### Example 6:

The gramine-platinum(IV) complex **e3** of the present example has the following structural formula:

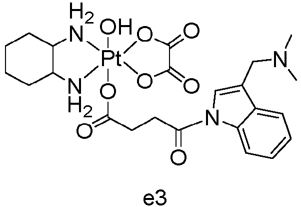

The preparation method (synthetic route) of the gramine-platinum(IV) complex of the present example was as follows:

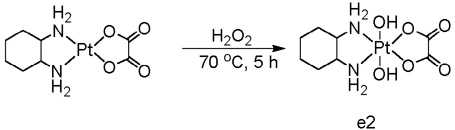

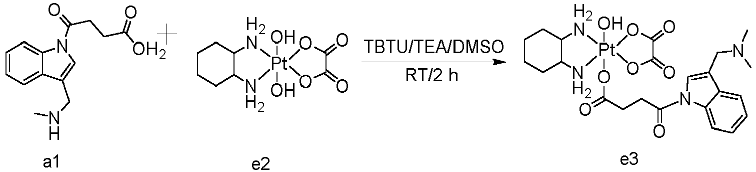

Step 1: Oxaliplatin (1.31 mg, 3.3 mmol) was weighed exactly, added to a round-bottom flask containing 2 mL of double distilled water and 10 mL of 30% hydrogen peroxide solution, and heated to reflux for 5 hours at 70°C in the dark. Subsequently, the heating was stopped, and the reaction solution was left to return to room temperature and then placed in a refrigerator at 4°C overnight. The precipitate was collected by centrifugation, washed twice with distilled water, anhydrous ethanol and anhydrous ether respectively, and dried under vacuum to obtain off-white crystalline Compound **e2** (0.87 g, yield 61.4%).

Step 2: Compound **a1** (63.4 mg, 0.23 mmol) was weighed exactly and dissolved in 1 mL of ultra dry DMSO. Then, TBTU (110.8 mg, 0.35 mmol) and TEA (48.3 µL, 0.35 mmol) were added successively and stirred for 15 minutes, and then, Compound **e2** (100 mg, 0.23 mmol) was added, and the mixture was left to react overnight under argon protection in the dark. When the reaction solution was in a clear state, the reaction was detected by TCL. After the completion of the reaction, an appropriate amount of dichloromethane was added to obtain a flocculent precipitate, which was collected by ultrasound and centrifugation. The precipitate was washed with dichloromethane and methanol respectively, and dried in a vacuum drying oven to obtain Compound **e3** as a light yellow solid (58.9 mg, yield 37.3%).

¹H NMR (400 MHz, DMSO-*d*₆): δ 8.38 (s, 1H), 8.33 (d, *J* = 7.4 Hz, 1H), 7.97 - 7.85 (m, 2H), 7.85 (d, *J* = 6.8 Hz, 1H), 7.46 (s, 1H), 7.36 (t, *J* = 7.5 Hz, 2H), 4.24 (s, 2H), 3.26 - 3.18 (m, 2H), 2.73 - 2.68 (m, 2H), 2.67 (s, 6H), 2.51 - 2.50 (m, 2H), 2.05 (d, *J* = 11.2 Hz, 2H), 1.49 (d, *J* = 7.6 Hz, 2H), 1.28 - 1.20 (m, 1H), 1.12 (t, *J* = 16.8 Hz, 3H).

¹³C NMR (101 MHz, DMSO-*d*₆): δ 180.50, 171.46, 163.94, 163.90, 135.05, 129.43, 125.06, 124.07, 123.44, 119.58, 115.72, 109.86, 61.24, 59.96, 51.09, 42.61, 31.34, 30.79, 30.73, 30.64, 23.64, 23.54.

HR-MS (m/z): calculated C₂₃H₃₂N₄O₈Pt (M + H)⁺ 688.19406; found: 688.19440.

### Example 7:

The gramine-platinum(IV) complex e of the present example has the following structural formula:

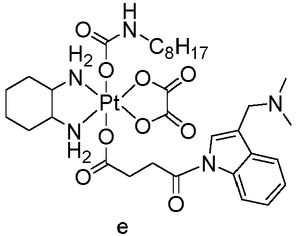

The preparation method (synthetic route) of the gramine-platinum(IV) complex of the present example was as follows:

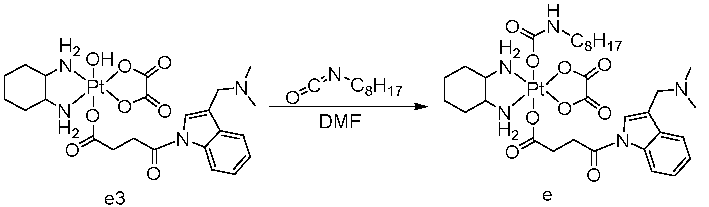

Compound **e3** (137.5 mg, 0.20 mmol) was weighed exactly and put in a 10 mL round-bottom flask containing 2 mL of ultra dry DMF. After complete dissolution, octyl isocyanate (50 µL) was added under stirring, and the mixture was left to react overnight at 60°C under argon protection in the dark. The reaction solution was monitored by TLC, and after the completion of the reaction, DMF was removed by rotary evaporation. The residue was purified by a preparative silica gel plate to obtain a white solid. The solid was washed with anhydrous ether and dried under vacuum to obtain Compound **e** (52.6 mg, yield 31.2%).

¹H NMR (400 MHz, DMSO-*d*₆): δ 8.30 (d, *J* = 8.0 Hz, 1H), 8.31 - 7.64 (m, 4H), 7.79 (s, 1H), 7.70 (d, *J* = 11.3 Hz, 1H), 7.28 (dt, *J* = 18.5, 6.9 Hz, 2H), 6.79 (s, 1H), 3.54 (s, 2H), 3.23 (s, 2H), 2.97 - 2.84 (m, 2H), 2.79 - 2.69 (m, 2H), 2.21 (s, 6H), 2.11 (s, 2H), 1.51 (s, 2H), 1.33 (s, 4H), 1.22 (s, 14H), 0.85 (t, *J* = 6.7 Hz, 3H).

¹³C NMR (101 MHz, DMSO-*d*₆): δ 179.42, 171.11, 164.44, 163.52, 163.31, 135.40, 130.16, 124.59, 124.47, 123.11, 120.00, 118.93, 115.67, 61.00, 60.62, 53.91, 45.05, 40.81, 31.25, 31.22, 31.08, 31.02, 29.61, 28.74, 28.69, 26.32, 26.26, 23.66, 23.35, 22.06, 13.91.

HR-MS (m/z): calculated C₃₂H₅₀N₅O₉Pt (M + H)⁺ 843.32508; found: 843.32526.

### Example 8:

The gramine-platinum(IV) complex **f** of the present example has the following structural formula:

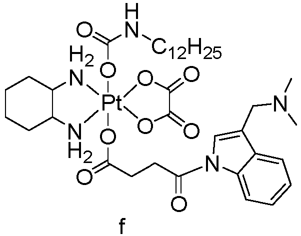

The preparation method (synthetic route) of the gramine-platinum(IV) complex of the present example was as follows:

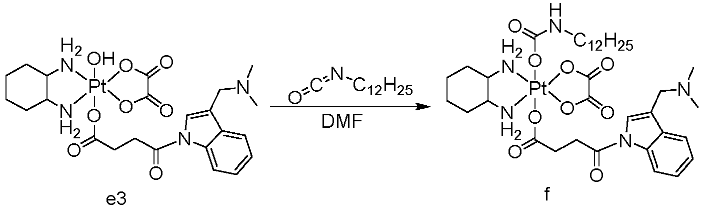

Compound **e3** (137.5 mg, 0.20 mmol) was weighed exactly and put in a 10 mL round-bottom flask containing 2 mL of ultra dry DMF. After complete dissolution, dodecyl isocyanate (50 µL) was added under stirring, and the mixture was left to react overnight at 60°C under argon protection in the dark. The reaction solution was monitored by TLC, and after the completion of the reaction, DMF was removed by rotary evaporation. The residue was purified by a preparative silica gel plate to obtain a white solid. The solid was washed with anhydrous ether and dried under vacuum to obtain Compound **f** (52.7 mg, yield 29.3%).

¹H NMR (400 MHz, DMSO-*d*₆): δ 8.30 (d, *J* = 8.0 Hz, 1H), 8.20 (s, 1H), 7.71 (d, *J* = 7.7 Hz, 1H), 7.65 (s, 1H), 7.48 (s, 1H), 7.28 (dt, *J* = 13.5, 7.7 Hz, 2H), 7.11 (s, 1H), 6.78 (s, 1H), 3.54 (s, 2H), 3.22 (s, 2H), 2.94 - 2.83 (m, 2H), 2.73 (t, *J* = 6.5 Hz, 2H), 2.20 (s, 6H), 2.11 (s, 2H), 1.50 (s, 2H), 1.33 (s, 4H), 1.23 (s, 20H), 0.85 (s, 3H).

¹³C NMR (101 MHz, DMSO-*d*₆): δ 179.43, 171.18, 164.37, 163.63, 163.42, 135.42, 130.19, 124.58, 124.48, 122.38, 118.93, 118.05, 115.73, 60.90, 60.51, 53.91, 45.09, 40.43, 31.27, 31.07, 30.95, 29.62, 29.05, 29.01, 28.98, 28.80, 28.73, 28.69, 26.29, 26.26, 23.63, 23.30, 22.06, 13.91.

HR-MS (m/z): calculated C₃₆H₅₈N₅O₉Pt (M + H)⁺ 899.38768; found: 899.38788.

### Example 9:

The gramine-platinum(IV) complex **g** of the present example has the following structural formula:

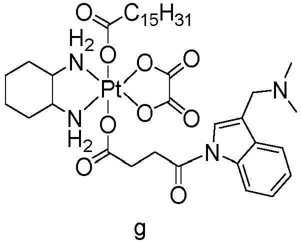

The preparation method (synthetic route) of the gramine-platinum(IV) complex of the present example was as follows:

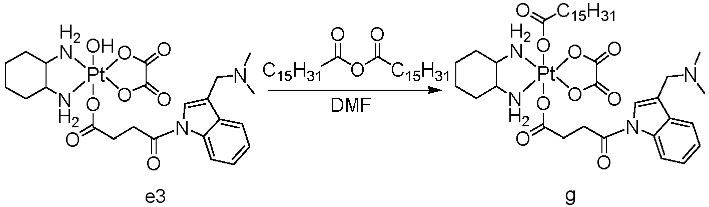

Compound **e3** (137.5 mg, 0.20 mmol) was weighed exactly and put in a 10 mL round-bottom flask containing 2 mL of ultra dry DMF. After complete dissolution, palmitic anhydride (200.1 mg, 0.40 mmol) was added under stirring, and the mixture was left to react overnight at 60°C under argon protection in the dark. The reaction solution was monitored by TCL, and after the completion of the reaction, DMF was removed by rotary evaporation. The residue was purified by a preparative silica gel plate to obtain a white solid. The solid was washed with anhydrous ether and dried under vacuum to obtain Compound **g** (53.3 mg, yield 28.9%).

¹H NMR (400 MHz, DMSO-*d*₆): δ 8.30 (d, *J =* 8.0 Hz, 1H), 8.28 (t, *J =* 16.9 Hz, 3H), 7.79 (s, 1H), 7.71 (d, *J* = 7.5 Hz, 1H), 7.32 - 7.23 (m, 2H), 6.77 (s, 1H), 3.55 (s, 2H), 2.94 - 2.84 (m, 2H), 2.73 (d, *J* = 5.9 Hz, 2H), 2.21 (s, 6H), 2.11 (s, 2H), 1.51 (s, 2H), 1.33 (s, 4H), 1.23 (s, 26H), 0.86 (d, *J* = 6.4 Hz, 3H).

¹³C NMR (101 MHz, DMSO-*d*₆): δ 179.49, 171.15, 164.38, 163.47, 135.40, 130.13, 124.58, 124.51, 123.10, 119.99, 118.83, 115.67, 60.75, 53.87, 45.01, 40.81, 31.41, 31.08, 30.93, 29.82, 29.79, 29.63, 29.58, 29.09, 29.06, 29.01, 28.80, 28.72, 28.67, 26.26, 23.71, 23.37, 22.10, 14.01.

HR-MS (m/z): calculated C₄₀H₆₆N₅O₉Pt (M + H)⁺ 926.41591; found: 926.57.

### Example 10: In vitro anti-tumor activity assay

The *in vitro* anti-tumor activity assay was conducted on the gramine-platinum(IV) complexes prepared in the above examples.

In this assay, the anti-tumor activity of the synthesized compounds was studied by MTT (3-(4,5-dimethylthiazolyl-2)-2,5-diphenyltetrazolium bromide) method. Eight kinds of cancer cells including MDA-MB-231 (human breast cancer cells), MCF-7 (human breast cancer cells), HCT-116 (human colon cancer cells), HeLa (human cervical cancer cells), FaDu (human pharyngeal squamous carcinoma cells), 5637 (human bladder cancer cells), OVCAR3 (human ovarian cancer cells), HCCC9810 (human liver cancer cells) and one normal human cell line LO2 (human liver cells) were used in this assay to perform experimental research on cytotoxicity. All cell lines were cultured in an incubator with 5% CO₂ and saturated humidity at 37°C. The specific experimental steps were as follows:
The cells in logarithmic growth phase were collected, counted, adjusted to a cell concentration of 3 × 10⁴ cells/mL, and then seeded into a 96-well plate at 100 µL/well. The blank group (Blank, pure medium group) and the control group (Control, no drug group) were set up. The plate was cultured overnight in a cell culture incubator. After adhesion, the compounds were diluted with culture medium and added to the first column of administration wells at a desired concentration for these wells, followed by the 2-fold serial dilution and administration in subsequent wells, and pipetted gently to mix well. After incubation for 72 hours, 10 µL of MTT solution (5 mg/mL) was added to each well. After cultivation in the incubator for another 4 hours, the supernatant was removed, and DMSO was added at 100 µL/well. After shaking thoroughly, the OD value was measured using a microplate-reader at λ = 570 nm. Three independently repeated experiments were carried out to ensure the reliability of the experimental results.

**Table 1. IC₅₀ values (µM) ^{β} of compounds after acting on cells for 72 hours**

| Compo und | MDA-MB-231 | MCF-7 | HCT-116 | HeLa | FaDu | 5637 | OVC RA3 | HCCC 9810 | LO2 |
|---|---|---|---|---|---|---|---|---|---|
| GM | >100 | >100 | >100 | >100 | -- | -- | -- | -- | >100 |
| **a3** | 3.73± 0.56 | 5.93± 1.77 | 6.18± 0.60 | 4.38± 1.22 | -- | -- | -- | -- | 7.24± 0.63 |
| | 1.65± 0.19 | 6.57± 1.72 | 15.73 ± 1.79 | 8.72± 0.29 | -- | -- | -- | -- | 4.01± 0.34 |
| **b** | 0.21± 0.00 | 1.14± 0.21 | 3.30± 0.93 | 2.44± 1.56 | -- | -- | -- | -- | 1.18± 0.06 |
| **c** | 0.07± 0.02 | 1.33± 0.18 | 0.26± 0.03 | 1.22± 0.03 | 0.03± 0.00 | 0.03± 0.00 | 0.04± 0.01 | 0.09± 0.00 | 0.92± 0.04 |
| **d** | 0.10± 0.01 | 0.41± 0.20 | 0.31± 0.03 | 0.78± 0.02 | -- | -- | -- | -- | 0.11± 0.00 |
| **e3** | 61.28 ± 8.95 | 43.89 ± 7.30 | 63.83 ± 1.03 | 49.03 ± 0.16 | -- | -- | -- | -- | 72.82 ± 9.92 |
| **e** | 2.83± 0.59 | 5.69± 0.03 | 60.33 ± 3.91 | 53.81 ± 6.93 | -- | -- | -- | -- | 1.33± 0.06 |
| **f** | 3.30± 0.22 | 7.14± 0.17 | 27.55 ± 0.89 | 42.53 ± 3.01 | -- | -- | -- | -- | 2.39± 1.44 |
| g | 0.10± 0.01 | 0.10± 0.01 | 0.99± 0.23 | 1.39± 0.22 | -- | -- | -- | -- | 0.17± 0.01 |
| CDDP | 15.77 ± 1.75 | 5.90± 0.63 | 8.79± 0.22 | 4.94± 0.31 | 1.57± 0.16 | 1.35± 0.05 | 1.93± 0.03 | 5.55± 0.58 | 3.99± 0.74 |
| OXP | 28.16 ± 3.18 | 17.49 ± 0.31 | 9.98± 0.70 | 14.63 ± 0.13 | -- | -- | -- | -- | 27.19 ± 7.51 |
| CDDP+ GM | 14.18 ± 0.11 | 5.06± 0.24 | 8.49± 0.17 | 4.85± 0.36 | -- | -- | -- | -- | 3.74± 0.61 |
| OXP+ GM | 25.27 ± 1.63 | 9.43± 0.45 | 7.02± 1.14 | 10.56 ± 0.34 | -- | -- | -- | -- | 23.42 ± 0.52 |
| FI^{γ} | 225.29 | 4.44 | 6.98 | 4.05 | 52.33 | 45 | 48.25 | 61.67 | 4.34 |

| | | | | | | | | | |
|---|---|---|---|---|---|---|---|---|---|
| Note: β represents an average value of multiple measurements; γ represents a value of IC₅₀ (CDDP)/IC₅₀ (Compound c); GM represents gramine; CDDP represents cisplatin; and OXP represents oxaliplatin. | | | | | | | | | |

As shown in Table 1, the anti-proliferation activity of the compounds against HeLa, MCF-7, MDA-MB-231, HCT-116, FaDu, 5637, OVCAR3, HCCC9810 and LO2 cells was detected. The IC₅₀ values of Compounds **a** to **g** were significantly lower than those of cisplatin, oxaliplatin, gramine, and the combined administration, demonstrating better anti-proliferation ability against cancer cells. Compared with the structure of Compound **a3,** Compound c has an additional hydrophobic chain of 12 carbons in the axial position. For the cancer cell lines studied, Compound c had IC₅₀ of 0.03-1.34 µM, and had the anti-proliferation activity higher than cisplatin by 225.29 times against the MDA-MB-231 cell line. In addition, Compound **c** had relatively low toxicity to LO2 cells and a selectivity index of 13.14 for the tumor cell line MDA-MB-231 and normal cell line LO2, whereas the selectivity index of cisplatin was only 0.25 (the selectivity index being defined as IC₅₀ (LO2)/IC₅₀ (MDA-MB-231)). In summary, Compound **c** has an anti-tumor effect with high efficacy and low toxicity.

### Example 11: Intracellular reduction experiment

The exploration of the intracellular release capacity of prodrugs is crucial for studying the mechanism of action of tetravalent platinum, which needs to be reduced to divalent platinum form *in vivo* to exert its effect. In order to investigate whether the gramine-platinum(IV) complexes can be reduced by reducing substances (glutathione, ascorbic acid, etc) in cells to release divalent platinum and exert drug sustained release effect, the intracellular reduction experiment was conducted. Taking Compound **c** as an example, the specific experimental steps were as follows:
1 × 10⁶ MDA-MB-231 cells were seeded into a 6-well plate. After the cell adherence, 100 µM of Compound **c** was applied to the cells and cultured for another 4 hours. The culture medium was discarded, and the cells were washed three times with PBS and centrifuged to remove PBS. The cells were resuspended with a certain amount of methanol and dichloromethane, transferred to a grinder and ground for 10 minutes until the cells were completely lysed. After being left to stand for a period of time, the supernatant was collected by centrifugation, and evaporated to remove the solvent at room temperature. Then, the solid was resuspended in 200 µL of methanol for chromatography, and detected using liquid chromatography under the following analysis conditions: UV detection wavelength of 260 nm, mobile phase of methanol and water (containing 0.1% formic acid), Venusil XBP C18 column (50 × 4.6 mm, 5 µm), Japan Shimadzu (LC-20A) high-performance liquid chromatography analyzer, gradient elution, methanol 5%-95% (0-10 min), 95% methanol for 25 min, flow rate of 1 mL/min.

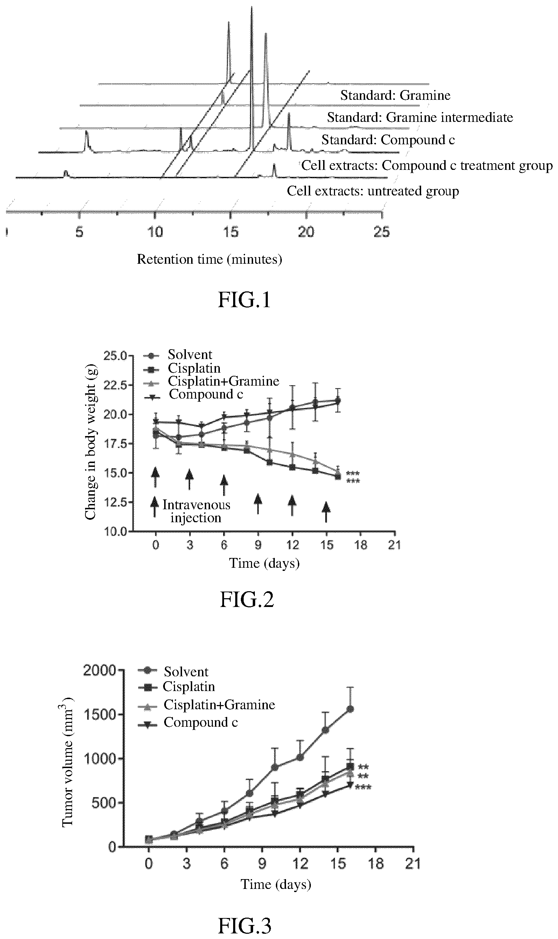

The HPLC detection results are shown in Figure 1. The bands from bottom to top represent the blank control group of cells without drug treatment, the intracellular extraction sample of Compound **c,** the standard of Compound **c,** the standard of Compound **a1** (GM-COOH, a gramine intermediate) and the standard of gramine (GM), respectively. Three absorption peaks can be observed on the band shown in the intracellular extraction sample of Compound c, wherein one peak appears at 9.8 min which was the same peak time as that of the GM standard, another peak appears at 10.7 min which was the same peak time as that of the GM-COOH standard, and the last peak appears at 12.5 min which was the same peak time as that of the standard of Compound **c,** demonstrating that Compound **c** can be released *in vivo.* Due to the drug treatment duration of only 4 hours, the release of GM and GM-COOH was relatively low, and Compound **c** would be further released under subsequent action. In summary, the gramine-platinum(IV) complexes synthesized in the present patent can be reduced in cells and have a sustained release effect.

### Example 12: Study on the in vivo anti-tumor activity of drugs (breast cancer model)

In order to investigate the anti-tumor effect of the gramine-platinum(IV) complex of the present disclosure, the study on the *in vivo* anti-tumor activity of the drugs was conducted. The specific steps were as follows:
Firstly, a 4T1-Luc tumor model was established using Balb/c female mice 4-5 weeks old. After the tumor volume reached 50-100 cm³, the mice were randomly divided into four groups, namely Solvent, Cisplatin, Cisplatin+Gramine, and Compound c groups, respectively. The drugs were formulated at a Pt concentration of 2.0 mg/kg, once every 3 days and six times in total, while the body weight and tumor volume of the mice were measured every 2 days. The mice were sacrificed and the organs (heart, liver, spleen, lung, kidney) and tumors thereof were excised. The organs and tumors were used for H&E staining, immunohistochemical staining, and ICP-MS determination of platinum content.

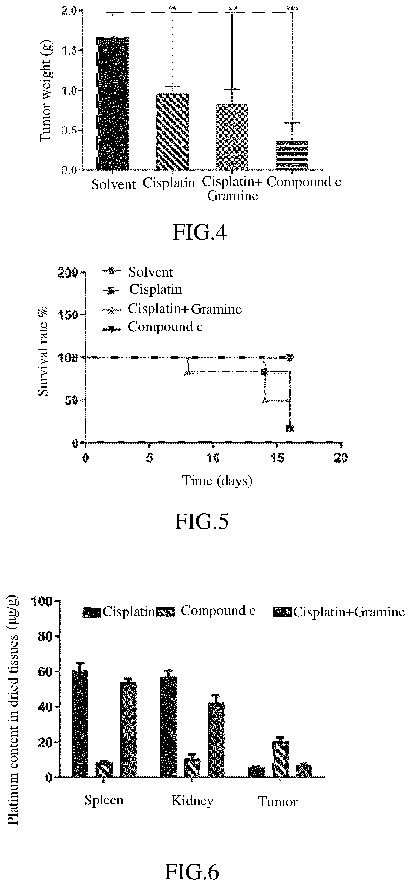

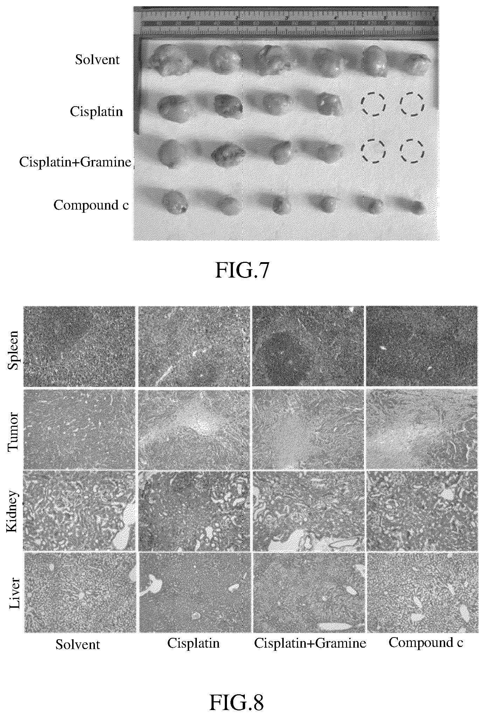

The experimental results are shown in Figures 2 to 9. From Figure 3 showing the tumor growth curves, Figure 4 showing the tumor weight and Figure 7 showing the final tumor image, it can be seen that Compound c has significantly inhibited tumor growth in comparison with the blank group, and has shown a significant improvement in the inhibitory effect compared with the cisplatin group and the combined administration group. Above all, compared with the cisplatin group and the combined administration group, Compound **c** has shown a significant decrease in toxicity to mice. From Figure 2 showing the body weight change of mice, it can be seen that the mice in the Compound **c** administration group have had substantially stable body weight without significant difference from that in the blank group, whereas the body weight of the mice in the cisplatin group and the combined administration group has shown a significant decline. The survival rate curves in Figure 5 are analyzed, showing that the mice in the Compound **c** group have had a final survival rate of 100%, whereas the mice in the cisplatin group and the combined administration group have had a final survival rate of only 16.67% (the dead mice including dead mice and mice with the body weight that has decreased by more than 20% of their initial body weight).

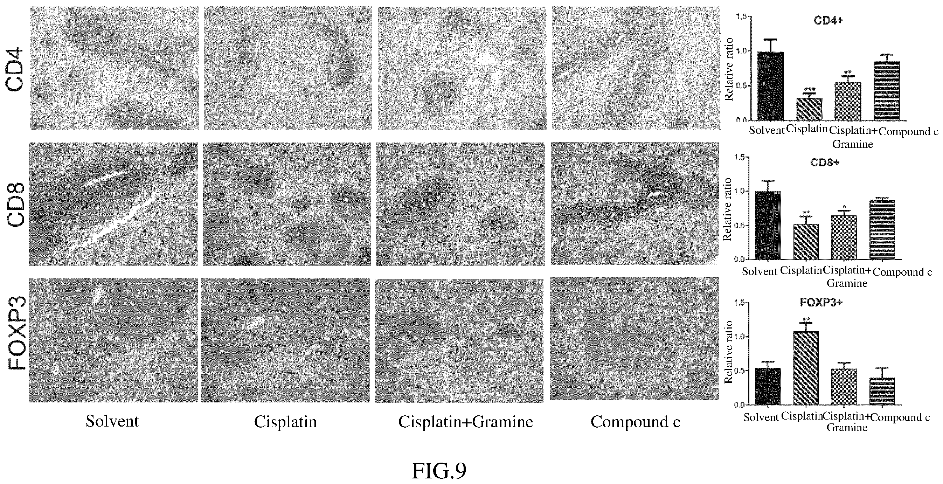

From the ICP-MS results in Figure 6, it can be seen that Compound **c** has significantly decreased the accumulation in the kidney and spleen in comparison with cisplatin. Cisplatin has caused renal toxicity as one of the most severe toxicities. The low accumulation of Compound **c** in the kidney makes it possible to effectively alleviate the toxic side effects of platinum drugs on the kidney. With H&E staining, it can be observed that both the cisplatin and Compound **c** groups have caused severe damage to tumor tissues. The Compound **c** group has resulted in minimal damage to mouse organs, whereas the cisplatin group has caused severe damage to the spleen, kidney, and liver, indicating a significant decrease in toxicity of Compound **c** compared with cisplatin. In addition, the immunohistochemistry results have shown that Compound **c** can activate immune regulation and enhance anti-tumor immune responses, for example, in Figure 9, increasing the number of CD4+ and CD8+ cells, while decreasing the number of Treg cells (Foxp3-labeled). The *in vivo* anti-tumor experiments above have demonstrated that Compound c exhibits significantly increased anti-tumor activity and significantly reduced toxic side effect compared with cisplatin, achieving the initial therapeutic goal of enhancing efficacy and reducing toxicity of new anti-tumor drugs.

### Example 13: In vitro determination of activity of inhibiting pancreatic cancer cells

This experiment was performed by CCK-8 (Cell Counting Kit-8) method to investigate the anti-tumor activity of Compound c as the gramine-platinum(IV) complex as well as cisplatin (CDDP) and oxaliplatin (OXP) as ligands. In this experiment, CCK-8 was used to study the proliferation activity of pancreatic cancer cells SU86.86, MIA-PaCa2, SW1990, CFPAC-1 and BxPC-3. All cell lines were cultured in an incubator with 5% CO₂ and saturated humidity at 37°C. The experimental steps were as follows:
The cells in logarithmic growth phase were collected, counted, adjusted to a cell concentration of 3 × 10⁴ cells/mL, and then seeded into a 96-well plate at 100 µL/well. The blank group (Blank, medium group without cells) and the control group (Control, medium group with cells) were set up. After the cells in the culture plate were cultured overnight in the incubator with saturated humidity at 37°C and adhered, the compounds were diluted with culture medium and added to the first column of administration wells at a desired concentration for these wells, followed by the 2-fold serial dilution and administration at 18 concentrations in total, and pipetted gently to mix well. After incubation for 72 hours, 10 µL of CCK8 solution (5 mg/mL) was added to each well. After cultivation in the incubator for another 4 hours, the OD value was measured using a microplate-reader at λ = 450 nm.

**Table 2. IC₅₀ values (µM) of compounds acting on cells**

| Compound | SU86.86 | MIA-PaCa2 | SW1990 | CFPAC-1 | BxPC-3 |
|---|---|---|---|---|---|
| CDDP | 126.70 | 500 | 500 | 603 | 420 |
| OXP | 8.02 | 2.00 | 2.80 | 200 | 1.37 |
| **c** | 0.07 | 0.01 | 0.31 | 0.89 | 0.03 |

| | | | | | |
|---|---|---|---|---|---|
| Note: CDDP represents cisplatin; and OXP represents oxaliplatin. | | | | | |

### Example 14: In vitro organoid activity assay

Compound c and clinical bivalent platinum as the control group (cisplatin or oxaliplatin) were selected to carry out the *in vitro* organoid activity assay in the organoid models of 2 cases of breast cancer and 2 cases of pancreatic cancer. The experimental steps were as follows:
Tumor samples were obtained from the patients undergoing surgery for breast cancer and pancreatic cancer, and digested with tumor dissociation kits. Tumor suspensions were collected and centrifuged to obtain tumor organoids. Complete culture medium for breast cancer or pancreatic cancer organoids was added to the organoid models which were cultured and passaged under the culture conditions of 5% CO₂ and saturated humidity at 37°C. The organoids in logarithmic growth phase were collected, counted, resuspended in Matrigel at an organoid concentration of 5 × 10⁴ cells/mL, and then seeded into a 96-well plate at 20 µL/well, followed by further addition of 80 µL of complete medium. The blank group (Blank, medium group without organoids) and the control group (Control, medium group with organoids) were set up. After the organoids were cultured overnight in an incubator with 5% CO₂ at 37°C and adhered, 200 µL of the culture medium containing the drug at a concentration of 20 µM was added to the first administration well, followed by the 2-fold serial dilution and administration, and pipetted gently to mix well. After incubation for 96 hours, representative photos were taken for each well using an inverted microscope, and 10 µL of CellTiter-Glo 3D Reagent (Promega) solution was added to each well to detect the organoid viability.

The detection results showed that Compound c exhibited significant inhibitory effect in all the organoid models of 2 cases of breast cancer and 2 cases of pancreatic cancer. Compound c had IC₅₀ values of 0.6359 µM and 1.65 µM in 2 cases of breast cancer respectively, and IC₅₀ values of 4.967 µM and 3.993 µM in 2 cases of pancreatic cancer respectively. However, in the organoid models of 2 cases of breast cancer, the IC₅₀ values of the cisplatin control group were all greater than 20 µM; and in the organoid models of 2 cases of pancreatic cancer, the IC₅₀ value of the oxaliplatin control group was 12.65 µM in one case, whereas greater than 20 µM in the other case. In summary, Compound c can exhibit highly effective anti-tumor effect in organoid models that are closer to the pathological conditions of tumors.

### Example 15: Study on the in vivo anti-tumor activity (pancreatic cancer PDX model)

Patient-derived xenografts (PDXs) were used. Fresh tumor tissue from a patient was treated and transplanted subcutaneously into the groin of immunodeficient NOD-SCID IL-2 receptor gamma null (NSG) mice. The xenograft tumor models similar to the original tumor characteristics were formed by means of the microenvironment provided by the mice. The successfully transplanted tumor was passaged for 3 passages or more in NSG mice to obtain mouse models for experiments. After the tumor volume reached 50-100 cm³, the mice were randomly divided into four groups, namely Solvent, Cisplatin, Oxaliplatin, and Compound c groups, respectively. The drugs were formulated at a Pt concentration of 3.0 mg/kg, once every 3 days and five times in total, while the body weight and tumor volume of the mice were measured every 3 days. The mice were sacrificed and the organs (heart, liver, spleen, lung, kidney) and tumors thereof were excised. The organs and tumors were used for ICP-MS determination of platinum content.

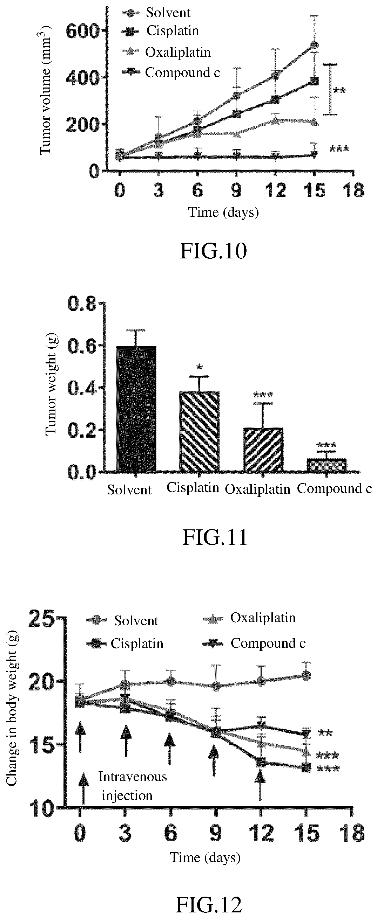

The detection results showed that, in terms of tumor growth as an indicator, Compound c exhibited significantly improved inhibitory effect compared with the solvent control group (PBS) and the positive control groups (cisplatin and oxaliplatin groups), and Compound c had a tumor inhibition rate of 97.44% at the treatment endpoint, whereas the cisplatin and oxaliplatin administration groups had tumor inhibition rates of only 32.68% and 68.82%. Meanwhile, compared with the cisplatin and oxaliplatin administration groups, Compound c also showed significant attenuation effect (see the curves of tumor volume change in Figure 10 and the tumor weight in Figure 11).

From the curves of body weight change of mice (Figure 12), it can be seen that the mice in the Compound c administration group showed weight recovery and stability from Day 9 post-treatment, which might be due to the fact that Compound c had activated the body's immune protection mechanism, whereas the body weight of the mice in the cisplatin and oxaliplatin administration groups showed a significant decline all the time.

In terms of the survival rate, the mice in the Compound c group had a final survival rate of 100%, whereas the cisplatin group had a final survival rate of only 40% and the oxaliplatin group had a survival rate of 60% (the dead mice including dead mice and mice with the body weight that has decreased by more than 20% of their initial body weight).

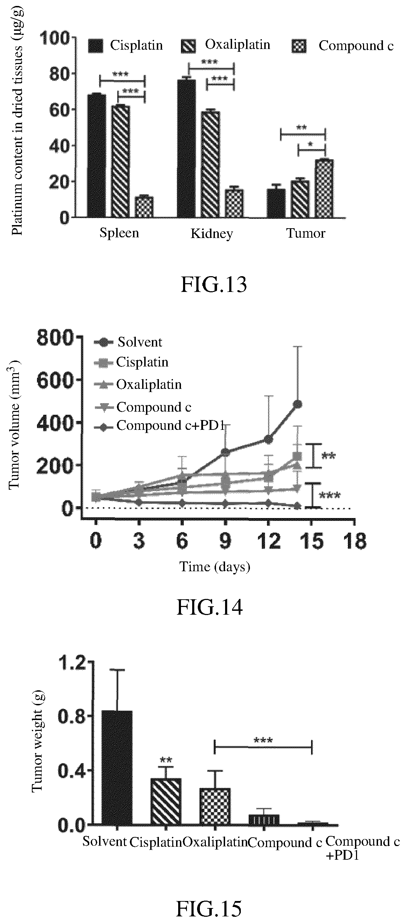

In addition, from the ICP-MS results in Figure 13, it can be seen that Compound c significantly decreased the accumulation in the kidney and spleen in comparison with cisplatin. Cisplatin caused renal toxicity as one of the most severe toxicities. The low accumulation of Compound c in the kidney enabled to effectively alleviate the toxic side effects of platinum drugs on the kidney. The *in vivo* anti-tumor experiments above have demonstrated that, in the pancreatic cancer model, Compound c exhibits significantly increased anti-tumor activity and significantly reduced toxic side effect compared with cisplatin and oxaliplatin, indicating that Compound c has a broad-spectrum anti-tumor effect.

### Example 16: Study on the in vivo anti-tumor activity (pancreatic cancer KPC model)

Firstly, a KPC tumor model was established using C57BL/6 female mice 4-5 weeks old. After the tumor volume reached 50-100 cm³, the mice were randomly divided into five groups, namely Solvent, Cisplatin, Oxaliplatin, Compound c, and Compound c + PD1 antibody (BioXcell BE0146-50MG InVivoMAb anti-mousePD-1(CD279)) groups, respectively. The drugs were formulated at a Pt concentration of 3.0 mg/kg, once every 3 days and five times in total, while the body weight and tumor volume of the mice were measured every 3 days. The mice were sacrificed and the organs (heart, liver, spleen, lung, kidney) and tumors thereof were excised. The tumors were used for flow cytometry to determine the content of immune cells.

The detection results showed that, in terms of tumor growth as an indicator, first of all, Compound c exhibited significantly improved inhibitory effect compared with the solvent control group (PBS) and the positive control groups (cisplatin and oxaliplatin groups), and Compound c had a tumor inhibition rate of 90.40% at the treatment endpoint, whereas the cisplatin and oxaliplatin administration groups had tumor inhibition rates of only 55.67% and 64.76%. More importantly, the Compound c+PD1 antibody combination group had a tumor inhibition rate of up to 107.88%, and exhibited significant tumor regression. Meanwhile, compared with the cisplatin and oxaliplatin administration groups, the Compound **c** group and the Compound c+PD1 antibody combination group showed significant attenuation effect (see the curves of tumor volume change in Figure 14 and the tumor weight in Figure 15).

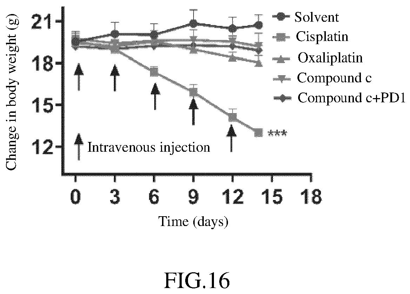

From the curves of body weight change of mice (Figure 16), it can be seen that the mice in the Compound **c** group and the Compound **c**+PD1 antibody combination group had substantially stable body weight without significant difference from that in the solvent control group, whereas the body weight of the mice in the oxaliplatin administration group shown a decline all the time, and the body weight of the mice in the cisplatin administration group shown a more significant decline.

In terms of the survival rate, the mice in both the Compound **c** group and the Compound **c**+PD1 antibody combination group had a final survival rate of 100%, whereas the mice in the cisplatin group all died finally (the dead mice including dead mice and mice with the body weight that has decreased by more than 20% of their initial body weight).

After treatment in combination with PD-1 antibody, compared with the solvent control group (PD-1: 15.7%) and the positive control groups of cisplatin (PD-1: 24.3%) and oxaliplatin (PD-1: 21.0%), the PD-1 level in the Compound c alone group decreased to 9.24%, confirming that Compound c alone could partially reverse the tumor immunosuppressive microenvironment. More importantly, the PD-1 level in the PD-1 antibody combination group significantly decreased to 1.96%, and the treatment with the combined administration almost completely reversed the immunosuppressive effect of PD-1. The above results together prove that the chemoimmunotherapy combining the effective dose of the gramine-platinum(IV) complex with the immunomodulatory drug has significant anti-tumor activity in pancreatic cancer.

The examples of the present disclosure have been explained in detail above, but are only the preferable examples of the present disclosure and cannot be considered as limiting the scope of implementation of the present disclosure. Various equivalent changes and improvements made according to the scope of the present disclosure shall still fall within the patent scope of the present disclosure.

## Claims

1. A gramine-platinum(IV) complex, **characterized in that** a gramine group is attached to a side in the axial direction of the tetravalent platinum coordination center, or a gramine group is attached to one side in the axial direction of the tetravalent platinum coordination center while a -NH-aliphatic chain group or an aliphatic chain group is attached to the other side in the axial direction of the tetravalent platinum coordination center;
in particular, the structure is shown in formula 1; 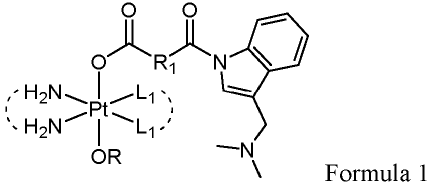
wherein, R is hydrogen atom, -C(O)-R₂, deuterium atom or C₁₋₆ alkyl; 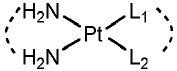 is selected from the group consisting of cisplatin, oxaliplatin, carboplatin, heptaplatin, nedaplatin, lobaplatin and miriplatin,
preferably, 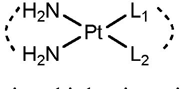 is cisplatin, oxaliplatin or carboplatin;
R₁ is -CₙH₂ₙ-, in which n is an integer and 1 ≤ n ≤ 6, and preferably, -CₙH₂ₙ- is a linear chain group;
R₂ is -CₘH₂ₘ₊₁ or -NH-CₘH₂ₘ₊₁, in which m is an integer and 1 ≤ m ≤ 20, and preferably, -CₘH₂ₘ₊₁ or -NH-CₘH₂ₘ₊₁ is a linear chain group.

2. The gramine-platinum(IV) complex according to claim 1, **characterized in that** the structure is shown in formula 1-1 or 1-2; 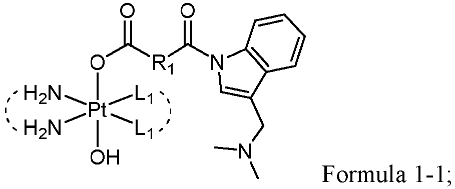 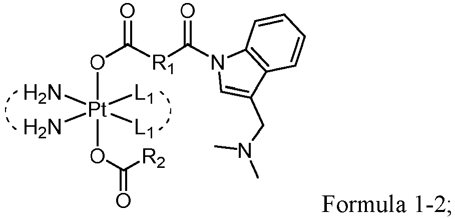 wherein, 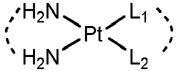
is selected from the group consisting of cisplatin, oxaliplatin, carboplatin, heptaplatin, nedaplatin, lobaplatin and miriplatin,
preferably, 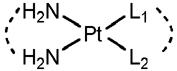 is cisplatin, oxaliplatin or carboplatin;
R₁ is -CₙH₂ₙ-, in which n is an integer and 1 ≤ n ≤ 6, and preferably, -CₙH₂ₙ- is a linear chain group;
R₂ is -CₘH₂ₘ₊₁ or -NH-CₘH₂ₘ₊₁, in which m is an integer and 1 ≤ m ≤ 20, and preferably, -CₘH₂ₘ₊₁ or -NH-CₘH₂ₘ₊₁ is a linear chain group.

3. The gramine-platinum(IV) complex according to claim 1 or 2, **characterized in that** -CₙH₂ₙ- is a linear chain group and n is 1 or 2.

4. The gramine-platinum(IV) complex according to any one of claims 1 to 3, **characterized in that** -CₘH₂ₘ₊₁ or -NH-CₘH₂ₘ₊₁ is a linear chain group with 6 ≤ m ≤ 17, preferably 6 ≤ m ≤ 15, and more preferably m being 6, 8, 12 or 15.

5. The gramine-platinum(IV) complex according to any one of claims 1 to 4, **characterized in that** it is represented by any one of structural formulas 2 to 10:

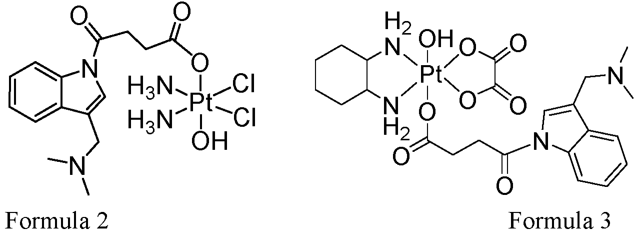

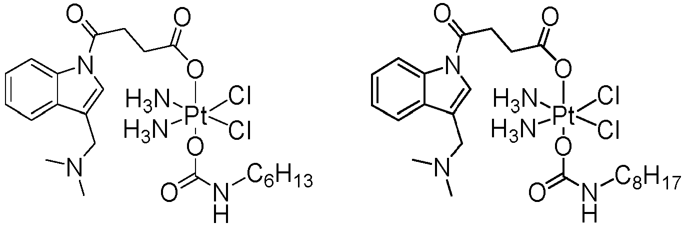

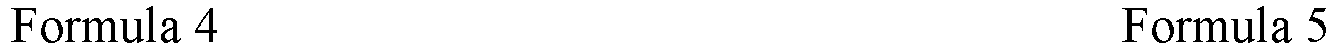

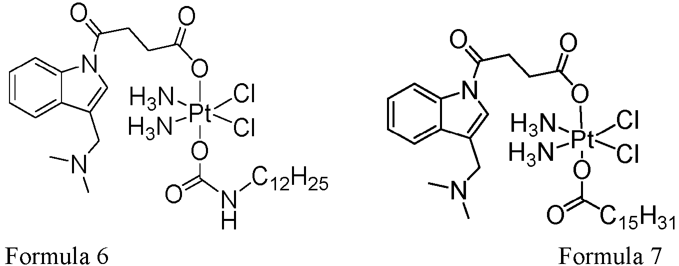

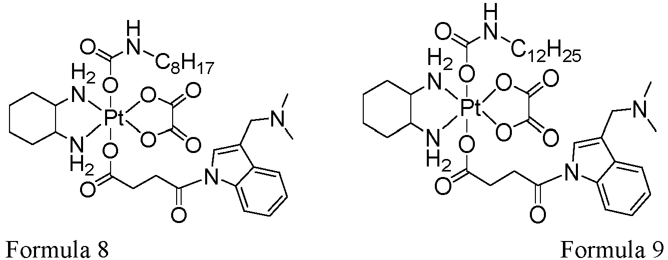

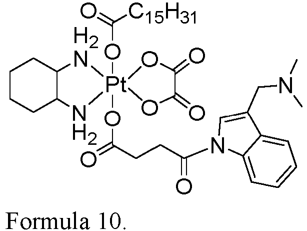

6. A method for preparing the gramine-platinum(IV) complex according to any one of claims 1 to 5, **characterized in that**:
a compound of formula 11 and a compound of formula 17 are subjected to the esterification reaction in the presence of a first condensing agent and a first acid binding agent to obtain the compound of formula 1; 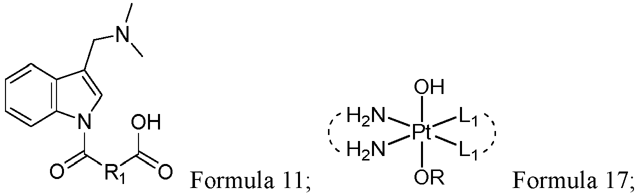
in particular, the compound of formula 11 and a compound of formula 12 are subjected to the esterification reaction in the presence of the first condensing agent and the first acid binding agent to obtain the compound of formula 1-1; 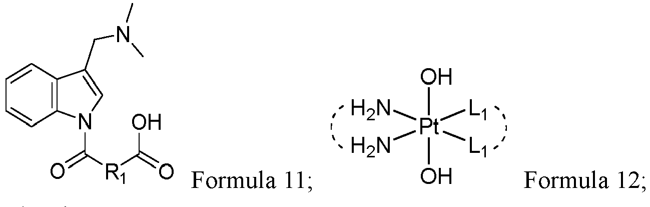
wherein,
R is hydrogen atom, -C(O)-R₂, deuterium atom or C₁₋₆ alkyl; 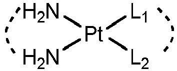 is selected from the group consisting of cisplatin, oxaliplatin, carboplatin, heptaplatin, nedaplatin, lobaplatin and miriplatin,
preferably, 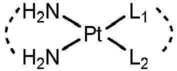 is cisplatin, oxaliplatin or carboplatin;
R₁ is -CₙH₂ₙ-, in which n is an integer and 1 ≤ n ≤ 6, and preferably, -CₙH₂ₙ- is a linear chain group;
especially, the first condensing agent is TBTU; the first acid binding agent is triethylamine; the ratio of raw materials in the reaction is as follows: the compound of formula 11: the compound of formula 17 or the compound of formula 12: the first condensing agent: the first acid binding agent is 1 to 1.2 : 1 : 1.2 to 2 : 1.2 to 2;
even, the reaction is carried out under the dark condition with inert gas protection.

7. The method for preparing the gramine-platinum(IV) complex according to claim 6, **characterized in that**: the compound of formula 1-1 and a compound of formula 13 or a compound of formula 14 are subjected to the esterification reaction to obtain the compound of formula 1-2; 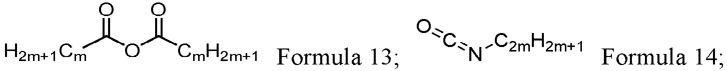 wherein,
m is an integer and 1 ≤ m ≤ 20, and preferably, -CₘH₂ₘ₊₁ is a linear chain group.

8. The method for preparing the gramine-platinum(IV) complex according to claim 6, **characterized in that**: gramine of formula 15 and a compound of formula 16 are subjected to the acylation reaction in the presence of a second condensing agent and a second acid binding agent to obtain the compound of formula 11; 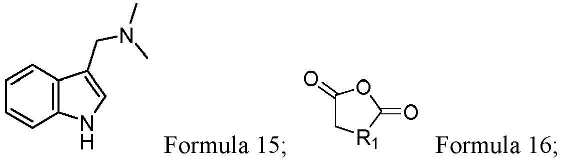 preferably, the second condensing agent is DMAP; and the second acid binding agent is triethylamine.

9. An isotopically substituted form of the gramine-platinum(IV) complex according to any one of claims 1 to 5, wherein preferably, the isotopically substituted form is a deuterated form.

10. A pharmaceutical composition comprising a therapeutically effective amount of at least one of the gramine-platinum(IV) complex according to any one of claims 1 to 5 or the isotopically substituted form according to claim 9, and a pharmaceutically acceptable excipient.

11. A pharmaceutical composition comprising the gramine-platinum(IV) complex according to any one of claims 1 to 5 or the isotopically substituted form according to claim 9, and an immunosuppressant and a pharmaceutically acceptable excipient.

12. A compound of formula 11 or a pharmaceutically acceptable salt thereof, 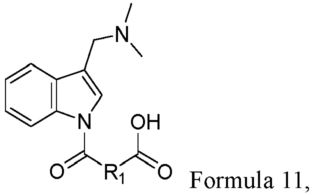
wherein R₁ is -CₙH₂ₙ-, in which n is an integer and 1 ≤ n ≤ 6, and preferably, -CₙH₂ₙ- is a linear chain group;
further, the compound of formula 11 is 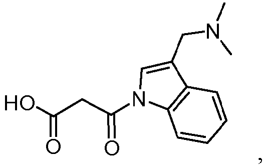 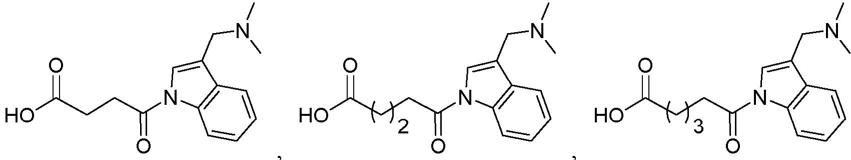
or

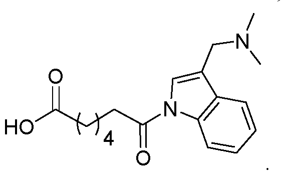

13. A use of the gramine-platinum(IV) complex according to any one of claims 1 to 5 or the pharmaceutical composition according to claim 10 or 11 or the compound of formula 11 or the pharmaceutically acceptable salt thereof according to claim 12 in the preparation of an anti-tumor medicament.

14. A use of the gramine-platinum(IV) complex according to any one of claims 1 to 5 or the isotopically substituted form according to claim 9 in combination with an immunosuppressant in the preparation of a medicament for treating a tumor.

15. The use according to claim 13 or 14, **characterized in that** the tumor is breast cancer, colorectal cancer, cervical cancer, head and neck cancer (such as pharyngeal squamous carcinoma), bladder cancer, ovarian cancer, pancreatic cancer, liver cancer or lung cancer.

16. The pharmaceutical composition according to claim 11 or the use according to claim 14, wherein the immunosuppressant is selected from the group consisting of an anti-PD-1 antibody or an antigen-binding fragment thereof, an anti-PD-L1 antibody or an antigen-binding fragment thereof, and an anti-CTLA-4 antibody or an antigen-binding fragment thereof,
the anti-PD-1 antibody or the antigen-binding fragment thereof is preferably Pembrolizumab, Nivolumab, Camrelizumab, Toripalimab, Tislelizuma, Cemiplimab, Sintilimab, CS-1003, HLX-10, SCT-I10A, sasanlimab, spartalizumab, MGD-013, retifanlimab, MEDI-0680, BAT-1306, MEDI-5752, LZM-009, JTX-4014, BI-754091, AK-104, BCD-217, balstilimab, AK-103 or cetrelimab,
the anti-PD-L1 antibody or the antigen-binding fragment thereof is preferably Atezolizumab, Avelumab, Durvalumab, Adebrelimab, Envafolimab, CS-1001, TQB-2450, KL-A167, IMC-001 or CX-072,
the anti-CTLA-4 antibody or the antigen-binding fragment thereof is preferably Ipilimumab or Tremelimumab.
